(19) Europäisches Patentamt
European Patent Office
Office européen des brevets

(11) **EP 1 488 797 B1**

(12) **EUROPEAN PATENT SPECIFICATION**

(45) Date of publication and mention
of the grant of the patent:
**23.04.2008 Bulletin 2008/17**

(51) Int Cl.:
*A23L 1/30* (2006.01)   *A23L 2/52* (2006.01)
*A23L 2/38* (2006.01)   *A23K 1/16* (2006.01)
*A61P 17/04* (2006.01)   *A61K 36/28* (2006.01)

(21) Application number: **01999380.7**

(22) Date of filing: **07.12.2001**

(86) International application number:
**PCT/JP2001/010741**

(87) International publication number:
**WO 2002/045733 (13.06.2002 Gazette 2002/24)**

(54) **ORAL PREPRATIONS HAVING ITCHING-RELIEVING OR ANTIPRURITIC ACTIVITY**

ORALE ZUBEREITUNGEN MIT JUCKREIZLINDERNDER ODER ANTIPRURITISCHER WIRKUNG

PREPARATIONS ORALES AYANT UNE ACTION DE SOULAGEMENT DES DEMANGEAISONS OU UN EFFET ANTIPRURIGINEUX

(84) Designated Contracting States:
**AT BE CH CY DE DK ES FI FR GB GR IE IT LI LU MC NL PT SE TR**
Designated Extension States:
**AL LT LV MK RO SI**

(30) Priority: **08.12.2000 JP 2000373693**

(43) Date of publication of application:
**22.12.2004 Bulletin 2004/52**

(73) Proprietor: **KYOWA HAKKO KOGYO CO., LTD.**
**Chiyoda-ku,**
**Tokyo 100-8185 (JP)**

(72) Inventors:
• **KOBAYASHI, Yoshinori**
**Tsukuba-shi,**
**Ibaraki 305-0841 (JP)**
• **NAKANO, Yumiko**
**Koganei-shi, Tokyo 184-0003 (JP)**
• **INAYAMA, Kiyoko**
**Kuwana-shi, Mie 511-0039 (JP)**
• **SAKAI, Akiko**
**Inashiki-gun,**
**Ibaraki 300-0398 (JP)**
• **KAMIYA, Toshikazu**
**Tsukuba-shi,**
**Ibaraki 305-0841 (JP)**

(74) Representative: **Holliday, Louise Caroline et al**
**D Young & Co**
**120 Holborn**
**London EC1N 2DY (GB)**

(56) References cited:
JP-A- 4 036 221      JP-A- 8 073 342
JP-A- 9 002 939      JP-A- 11 029 460
JP-A- 2000 044 481

• PATENT ABSTRACTS OF JAPAN vol. 018, no. 334 (C-1216), 24 June 1994 (1994-06-24) -& JP 06 078727 A (SOGO INSATSU KOGEI KK), 22 March 1994 (1994-03-22)
• TOSHIHIKO TSUJI ET AL.: "Inhibitory effect of plant extracts on degranulation and histamine release from purified rat mast cells" JOURNAL OF THE SOCIETY OF COSMETIC CHEMISTS OF JAPAN - NIHON KESHOHIN GIJUTSUSHAKAI KAISHI, vol. 25, no. 4, 1992, pages 246-253, XP008043817 JPNIHON KESHOHIN GIJUTSUSHAKAI, TOKYO
• DATABASE WPI Section Ch, Week 198525 Derwent Publications Ltd., London, GB; Class B04, AN 1985-149130 XP002319704 -& JP 60 081129 A (YAMADA T) 9 May 1985 (1985-05-09)
• T. MILLER ET AL.: "Effects of some components of the essential oil of chamomile, Chamomilla recutita, on histamine release from rat mast cells" PLANTA MEDICA, vol. 62, no. 1, 1996, pages 60-61, XP008043836 DETHIEME, STUTTGART
• HO CHEONG ET AL.: "Desacetylmatricarin, an anti-allergic component from Taraxacum platycarpum" PLANTA MEDICA, vol. 64, no. 6, 1998, pages 577-578, XP008043818 DETHIEME, STUTTGART

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- C. ANDERSON ET AL.: 'Evaluation of massage with essential oil on childhood atopic eczema' PHYTOTHERAPY RESEARCH vol. 14, 2000, pages 452 - 456, XP002909307

- M. RODRIGUEZ-SERNA, J.M. ET AL.: 'Allergic and systemic contact dermatitis from matricaria chamomilla tea' vol. 39, no. 4, 1998, pages 192 - 193, XP002909308

## Description

### Technical Field

The present invention relates to the use of a plant body or extract thereon in the preparation of a medicament for treating pruritis. The medicament may be in the form of an oral preparation, food, drink or feed having an itch-relieving or antipruritic activity.

### Background Art

Itch is thought as being perceived through sensory nerves. In other words, it is said that itch is a feeling or a deformation of pain on the cutaneous mucosa, which is different from pain such as a localized or diffuse pricking and is emerged spontaneously. When a mild and persistent stimulation is yielded, the stimulation is transmitted via the spinal dorsal roots, the spinal anterolateral axons and the thalamus from a sensory nerve to the cerebral cortex where it is recognized as an itch.

Itch is thought to emerge when various causes mildly as well as persistently stimulate the end plate of the dermal sensory nerve, especially the C-fiber nerve receptor distributed in the subepidermal plexus nervorum. Alternatively, some itches are regarded to be central, which are recognized by the thalamus or cortex of the cerebral center (see Medical Dictionary, section "Pruritus", NANZANDO Co., Ltd., 1998).

As an anti-pruritus method, i.e. a method to treat itch, a generally known method is the one in which a primary disease is treated while itch is controlled with an antihistamine agent or a suppressor of chemical mediator release. Besides, as for an itch therapy, for example, administration of the adrenal cortex hormone to suppress inflammation, administration of sedatives to suppress psychogenic causes, application of moisturizers for dry skin, and the like are known (see Medical Dictionary, section "Itch", NANZANDO Co., Ltd., 1998).

On the other hand, in the case of a diffuse itch, especially in the case of itch derived from atopic dermatitis, an intractable itch is known to remain which cannot be overcome by the use of anti-histamine agents. Dry skin or inflammation is sometimes triggered when the affected area is scratched in order to resist itch, and this is thought to be a significant cause for the further exacerbation of the symptoms as well as making the situation chronic.

Atopic dermatitis is known to be easily exacerbated by external stimuli such as perspiration, scratching, friction, and the like (Yuko HIGAKI, Appendix to Experimental Medicine, Medical Term Library, "Allergy", pp.128-129, 1996). Itch is, therefore, the most crucial target of the therapy for the disease (Kiyoshi NISHIOKA, "Text for Atopic Dermatitis", pp.54-57, Nankodo, 1995).

Antihistamine/anti-allergy agents, anti-inflammation agents, moisturizers, antibacterial agents, anti-oxidants, etc. are known as therapeutic agents for atopic dermatitis.

However, pharmaceuticals or food capable of fully suppressing itch caused by atopic dermatitis are not yet known.

The following pharmacological actions and effect-efficacy are known for German chamomile (Matricaria chamomilla LINNAEUS; also called Matricaria recutita L. or Chamomilla recutita (L.) RAUSCHERT) which is a plant that belongs to the genus Matricaria of Compositae, and Roman chamomile (Anthemis nobilis L.; also called Chamaemelum nobile (L.) ALLIONI) which is a plant that belongs to the genus Anthemis of Compositae.

There is an old custom in Europe to drink a chamomile tea served by pouring hot water onto chamomile flowers, for common cold, headache and diarrhea. In China, chamomile is called mother chrysanthemum or European medicinal chrysanthemum, and its flowers and whole plant are used for common cold and rheumatic pains (Makino's Illustrated Encyclopedia of Japanese and Chinese Medical Plants in Colour, General editor; Hiroshi MITSUHASHI, p. 568, Hokuryukan Co., Ltd.) In particular, dried flower heads are used as anti-inflammatory agents, antispasmodic agents and carminative agents. The flower heads are effective for stomach disorders as an aromatic bitter stomachic and are known to increase the appetite by promoting secretion in the gall bladder and stomach as well as acting as a vomiting agent when taken a large amount.

A healthy drink is known which contains a chamomile extract and is effective for the promotion of health (Japanese Patent No. 2124237). Further, as for oral preparations, for example, an anti-inflammatory composition comprising powder or extract of herbs such as chamomile and the like, having inhibitory activity against 3α-hydroxysteroid dehydrogenase or hyaluronidase, is known (Japanese Published Unexamined Patent Application No. 145430/91).

On the other hand, a chamomile extract is a commonly used herbal drug used for cosmetics, and is respectively used for shampoo, hair rinse and the like for giving shininess and body to hair and preventing loss of hair, for basic skin care products such as skin cream, skin lotion, etc. for sedating skin which easily suffers from dryness or inflammation, and for bath preparations for promoting cure of injuries and suppressing itch derived from hemorrhoids or chronic eczema ('95 Application of Medical Herb, pp.90-91, CMC Publishing Co., Ltd., 1995).

Further, as for external preparations which comprise chamomile extracts, the following, for example, and the like are known: a bath agent having suppressing actions on antibody-production and skin-softening action, which is suitable for easing the symptoms of atopic dermatitis or the like (Japanese Published Unexamined Patent Application No. 100236/97) and; a hair restorer comprising a chamomile extract and cepharanthin as active ingredients, which is effective for preventing dandruff and itch (Japanese Published Examined Patent Application No. 6525/94).

JP06078727A relates to a heath beverage comprising extracts of chamomile, carrot, Hanbi, Coptis japonica, Kawara-take, liquorice and plum.

Tsuji et al (J. Soc. Cosmet. Chem. Japan (1992) 25:1-15) is a study investigating the inhibitory effect of extracts of seven kinds of plants on degranulation and histamine release from rat cells *in vitro*.

Anderson et al (Phytotherapy Research (2000) 14 :452-456) is a study to investigate whether massage with essential oils (aromatherapy) used as a complementary therapy, in conjunction with normal medical treatment, helps to alleviate the symptoms of childhood atopic eczema.

However, itch-relieving or antipruritic activity of chamomile by oral intake is yet unknown.

**Disclosure of the Invention**

The object of the present invention is to provide oral preparations, foods, drinks, feeds, food additives or feed additives having itch-relieving or antipruritic activity, for example, having activities to ease chronic and intractable itches such as skin itch from atopic dermatitis, eye itch from pollinosis, skin itch from dry skin or the like and to prevent the exacerbation of the symptoms brought by scratching.

The present invention relates to (1) to (6) mentioned below.

(1) The use of a plant body of a plant that belongs to the genus *Matricaria* or *Anthemis* or of an extract of the plant body in the preparation of a medicament for oral administration for treating pruritis.
(2) The use according to (1), wherein the plant that belongs to the genus *Matricaria* or *Anthemis* is chamomile.
(3) The use according to (1) or (2), wherein the plant that belongs to the genus *Matricaria* or *Anthemis* is German chamomile (*Matricaria chamomilla* LINNAEUS) or Roman chamomile (*Anthemis nobilis* L.).
(4) The use according to (1) or (2), wherein the oral medicament is for the treatment of itch derived from atopic dermatitis or pollinosis.
(5) The use according to (1), (2), (3) or (4), wherein the oral medicament is a food, drink or feed.
(6) The use according to (1), (2) (3) or (4), wherein the oral medicament is a food additive or a feed additive.

Although any plant that belongs to the genus *Matricaria* or *Anthemis* may be used in the present invention, plants classified as chamomile are preferably used.

As for plants that belong to the genus *Matricaria*, chamomile such as *Matricaria chamomilla* LINNAEUS, *Matricaria inodora* L., *Matricaria matricarioides* (Less.) Porter, *Matricaria tetragonosperma* (Fr. Schm.) Hara et Kitam. and the like are exemplified.

As for plants that belong to the genus Anthemis, chamomile such as *Anthemis nobilis* L., *Anthemis arvensis* L., *Anthemis tinctoria* L. and the like are exemplified.

Among the plants that belong to the genus *Matricaria* or *Anthemis*, *Matricaria chamomilla* LINNAEUS and *Anthemis* nobilis L. are preferred for use, and *Matricaria chamomilla* LINNAEUS is particularly preferred for use.

Whole of a wild plant body, of a plant body obtained by cultivation, of a plant body obtained by the culture such as a tissue culture, or organs such as a flower, fruit, seed, leaf, branch, stem, root and the like may be used in the present invention. However, a flower, particularly a flower head, is preferably used. Cultured cells, callus, organs, etc. may also be used as a plant body.

A plant body in the context of the present invention may be used untreated or used as a treated plant body which has undergone a physicochemical or biological treatment.

Physicochemical treatments include a drying treatment such as sun-dry, wind-dry, freeze-dry and the like, crushing treatment such as blending, homogenizing, ball-milling and the like, and the physicochemically treated substances include a dry-treated substance, a crush-treated substance, and the like.

Biological treatments include a fermentation treatment using bacteria, yeast, and the like, and the biologically treated substances include a fermented substance and the like.

As for an extract of a plant body for use in the present invention, the extract may be obtained from the plant body by a process of extracting a substance such as a solvent extraction, a supercritical fluid extraction and the like, are exemplified.

Any solvent capable of extracting an ingredient having itch-relieving or antipruritic activity may be used as the solvent for the solvent extraction and, for instance, an aqueous medium, an organic solvent and the like may be used. In addition, a solvent may be used alone or used as a mixed solvent wherein multiple solvents are combined.

Examples of an aqueous medium include water, purified water, deionized water, distilled water, and the like

Any solvent with which a substance having itch-relieving or antiprurituc activity is extracted may be used as an organic solvent, and the examples include: a univalent aliphatic alcohol such as methanol, ethanol, 1-butanol, 1-propanol, 2-butanol and 2-propanol; a bivalent aliphatic alcohol such as ethylene glycol and propylene glycol; a bivalent alcohol such as glycerin; alkyl acetate such as methyl acetate and ethyl acetate; an aliphatic ketone such as acetone and ethyl methyl ketone; an aliphatic ether such as dimethyl ether and diethyl ether; an aliphatic hydrocarbon such as hexane, heptane and petroleum ether; an alicyclic hydrocarbon such as cyclohexane; an aromatic hydrocarbon such as toluene and benzene; an aliphatic hydrocarbon halide such as chloroform, dichloromethane, 1,1,1,2-tetrafluoroethane and 1,1,2-trichloroethene; an edible fat and oil such as sesame oil, corn oil, olive oil and cotton seed oil; a liquid aliphatic hydrocarbon such as methane, ethane, propane, propylene, butane and buthylene; dimethylsulfoxide, and the like.

An alkyl acetate such as ethyl acetate and the like, a univalent aliphatic alcohol such as ethanol and the like, aliphatic ketone such as acetone and the like, are preferred as an organic solvent, and ethanol is more preferred for use in view of oral administration.

As for a mixed solvent, a hydrated univalent alcohol, for instance, is preferred and water content is preferably equal to 90% (v/v) or below, where 50% (v/v) or below is more preferred.

A device used in an ordinary solvent extraction is employed for the solvent extraction, including an agitator, an ultrasonic generator, a reflux extractor, Soxhlet extractor, and the like.

There is no particular limitation to a solvent amount used for the solvent extraction and, for example, 0.1 to 10000 parts by weight (w/t parts) of a solvent to 1 w/t part of a plant body is used. Preferably 1 to 1000 w/t parts, more preferably 5 to 100 w/t parts of solvents to 1 w/t part of a plant body is used. Although there is no particular limitation to the extraction temperature as long as the temperature is equal to or higher than the melting point of a solvent and equal to or lower than the boiling point of a solvent, 0 to 100°C is preferred for aqueous medium, where 20 to 80°C is more preferred, and 0 to 1000° C is preferred for organic solvents, where 20 to 80°C is more preferred. There is no particular limitation to the time for extracting, however, 1 minute to 1 year is preferred and 30 minutes to 1 week is more preferred.

After the solvent extraction process is completed, the extract liquid obtained may undergo a solid-liquid separation method such as a precipitation separation, a cake filtration, a clarification filtration, a centrifugal filtration, a centrifugal precipitation, compression, separation, a filter press and the like, among which filtration is preferred. Then the extract liquid thus obtained may be used as an extract. Moreover, an extraction residue obtained from the solid-liquid separation method may further be extracted with an extraction solvent and the resultant extract may be used as an extract.

When performing a supercritical fluid extraction (occasionally referred to merely as a supercritical extraction), an extractor fluid may be used alone or an extractor fluid to which an entrainer is mixed may be used.

Any supercritical fluid may be used as an extractor fluid from among carbon dioxide, ammonium, methanol, ethanol, isopropyl alcohol, ethane, propane, butane, pentane, hexane, dimethylbutane, benzene, dichlorodifluoromethane, dichlorofluoromethane, trichlorofluoromethane, dichlorotetrafluoromethane, chlorotrifluoromethane, diethylether, ethylmethylether, hexane, dinitrogen monoxide and the like, but a supercritical carbon dioxide is preferably used.

In the present invention, a supercritical fluid (also referred to as a supercritical gas) is a fluid in a state of beyond the supercritical temperature and supercritical pressure.

Any entrainer including a univalent aliphatic alcohol such as methanol and ethanol, water, a hydrated univalent aliphatic alcohol, acetone, hexane and the like may be used, however, ethanol is particularly preferred for use. An entrainer amount to be added is preferably 0.1 to 50% (w/w) and 1 to 20% (w/w) is more preferred.

There is no particular limitation to an extractor fluid amount used for the supercritical fluid extraction and, for example, 5 to 1000 w/t parts, preferably 20 to 50 w/t parts of an extractor fluid in integrated volume to 1 w/t part of a plant body is used. An extractor fluid may be circulated if necessary.

There is no particular limitation to an entrainer amount used for a supercritical fluid extraction and, for example, 0.001 to 100 w/t parts, preferably 1 to 20 w/t parts in integrated volume to 1 w/t part of supercritical fluid, is used.

A supercritical fluid extractor is used for the supercritical fluid extraction.

Temperature and pressure of an extraction vessel for the supercritical fluid extraction must be beyond the supercritical temperature and supercritical pressure respectively, but there is no limitation to the upper limit.

The supercritical temperature and supercritical pressure differ depending on compounds. In the case of carbon dioxide for example, the temperature of the extraction vessel is preferably 31.06 to 1000° C, where 31.1 to 60° C is more preferred, and the extraction vessel pressure is preferably 72.9 to 5000 atm, where 75 to 1000 atm is more preferred. Although there is no particular limitation to the separation vessel pressure, 1 to 5000 atm is preferable and 1 to 1000 atm is more preferred.

There is no particular limitation to the temperature of a separation vessel used in a supercritical fluid extraction, however, -273.16 to 1000°C is preferred and -80 to 200° C is more preferred. Although there is no specific limitation to the time for extracting, 1 minute to 100 hours is preferred and 20 minutes to 5 hours is more preferred.

After the supercritical fluid extraction is completed, the extract liquid can be obtained by altering temperature or pressure of the separation vessel, a filling agent or the like. As for a method for separating the extract, an isothermal/decompression method, an isothermal/gas-liquid separation, an isobaric/heating method, an isobaric/cooling method, an adsorption method or the like are exemplified. After the separation operation is completed, an extract can be obtained as a solid or liquid form in the separation vessel or as an entrainer solution when an entrainer is used.

An extract obtained from a plant body by an extraction method such as a solvent extraction method, a supercritical fluid separation method or the like, may further be treated by a solid-liquid separation method, a concentration or drying process, a purification process and the like. Then the resulting extract can also be used as the extract of a plant body in the present invention.

As for a solid-liquid separation method, a precipitation separation, a cake filtration, a clarification filtration, a centrifugal filtration, a centrifugal precipitation, a compression separation, a filter pressing or the like are exemplified.

As for a concentration or drying method, the examples include drying methods such as membrane drying methods including a thermal concentration, a freeze concentration, a reverse osmosis concentration, a decompression concentration, a freeze drying, a natural drying, a hot-air drying, a wind drying, a blast drying, a spray drying, a reduced-pressure drying, a sun drying, a vacuum drying, a spray drying, a fluidized-bed drying, a foam-layer drying and a drum dryer; an ultrasonic drying method; a microwave drying method, and the like. A decompression concentration method, a spray drying method and a freeze drying method are preferably employed.

After the above-mentioned extract is dissolved in a solvent as required, the concentration of an ingredient having itch-relieving or antipruritic activity in the extract may be increased or the unnecessary substances may be removed by the purification methods of a membrane separation, a liquid membrane separation, a solvent distribution, fractionating or the like.

As for a solvent distribution method, a liquid-liquid extraction method using an aqueous medium and an organic solvent, and an aqueous two-phase distribution method using a colloid-containing liquid are exemplified, which can be performed by using a separatory funnel or a counter-current distribution equipment or the like.

As for a fractionating method, examples include a partition chromatography, an adsorption chromatography, an ion-exchange chromatography, an affinity chromatography, a gel chromatography, dialysis and the like. The method preferably used among these is an adsorption chromatography in which a hydrophobic adsorption resin such as HP-20 DIAION (Mitsubishi Chemical Corporation), LH-20 Sephadex (Pharmacia), COSMOSIL 140 C18-OPN (Nacalai Tesque, Inc.) and the like, and an affinity adsorption resin such as SILICAGEL 60 (Nacalai Tesque, Inc.) and the like, are used as a carrier and an aqueous medium, methanol, acetone, ethyl acetate, chloroform, toluene, hexane and the like, are used as a solvent.

When preparing the extract of a plant body for use in the present invention, an anti-oxidant, a preservative, for instance, may be added and the heating temperature may be adjusted in order not to deactivate an ingredient having itch-relieving or antiprurutic activity.

An oral preparation for use in the present invention contains a plant body of the present invention or the extract of the plant body as an active ingredient and may also contain one or more pharmacologically acceptable carriers if necessary and may further contain other therapeutically active ingredient if necessary.

An oral preparation for use in the present invention is produced by mixing, if necessary, a plant body of the present invention or the extract of the plant body with a carrier in accordance with any optional method well known in the field of pharmaceutical technology.

Examples of dosage forms for administration include: tablets, powder agents, granules, pills, capsules, suspensions, emulsions, elixirs, syrups, liquid agents, infusions/decoctions, extracts, tinctures, fluid extracts and the like. Infusions/decoctions, extracts, tinctures, fluid extracts and the like are preferably used as oral preparations, because these may be prepared either as an extract per se or by concentrating the extract which is extracted from a plant body of a plant that belongs to the genus Matricaria or Anthemis with, for example, water, ethanol, a mixture of water and ethanol, a supercritical carbondioxide and the like.

When formulating oral preparations for use in the present invention, additives may be used such as excipients, binders, disintegrators, lubricants, dispersions, suspensions, emulsifiers, diluents, buffers, anti-oxidants, bacteria-suppressants and the like.

When a dosage form of an oral preparation is a tablet, a powder agent, a granule and the like, the oral preparation can be formulated by adding saccharides such as lactose, white sugar, glucose, sucrose, mannitol and sorbitol; starches such as potatoes, wheat and corns;inorganic materials such as calcium carbonate, calcium sulfate, sodium hydrogencarbonate and sodium chloride; excipients from plant powders or the like such as licorice powder and gentian powder; disintegrators such as starch, agar, gelatin powder, crystal cellulose, carmellose sodium, carmellose calcium, calcium carbonate, sodium hydrogencarbonate and sodium alginate; lubricants such as magnesium stearate, talc, hydrogen-supplemented vegetable oil, macrogol and silicon oil; binders such as polyvinyl alcohol, hydroxypropyl cellulose, methyl cellulose, ethyl cellulose, carmellose, gelatin and a starch glue liquid; surfactants such as fatty acid ester; plasticizers such as glycerin, and the like.

When a dosage form of an oral preparation is in the form of liquid preparations such as syrup or the like, the oral preparation can be formulated by adding water; saccharides such as sucrose, sorbitol and fructose; glycols such as polyethylene glycol and propylene glycol; oils such as sesame oil, olive oil and soy oil; antiseptics such as ester p-hydroxybenzoate; flavors such as strawberry flavor and peppermint flavor, and the like.

Although the dose of oral preparations for use in the present invention for human differs depending on the administration form, the extent of symptoms, age and weight of a patient or the like, a dose per day for an adult is usually 0.1 to 5000 g in dry weight, preferably 1 to 500 g, of a plant body of a plant that belongs to the genus Matricaria or Anthemis, which is administered either once or multiple times a day. In the case the extract of a plant body is used, an extract obtained from a plant body with dry weight within the above-mentioned range may be administered. There is no particular limitation to the period of administration, however, the period is usually from 1 day to 1 year, preferably 1 week to 3 months.

Although the dose of oral preparations for use in the present invention for animals differs depending on the administration form, animal variants to be administered, age and weight of the animal or the like, it is usually 0.1 mg to 150 g/kg in dry weight, preferably 1 mg to 15 g/kg, of a plant body of a plant that belongs to the genus Matricaria or Anthemis, which is administered either once or multiple times a day. In the case where the extract of a plant body is used, an extract obtained from a plant body with dry weight within the above-mentioned range may be administered. There is no particular limitation to the period of administration, however, the period is usually from 1 day to 1 year, preferably 1 week to 3 months.

Oral preparations for use in the present invention may be simultaneously administered with other anti-inflammatory agents such as steroids, anti-histamine agents, anti-allergic agents and the like. Especially when simultaneously used along with steroids, a marked itch-relieving or antipruritic effect can be conferred.

Glucosteroids such as predonisolone, cortisol, dexamethasone and betamethasone, and saline steroids such as fludrocortisone and aldosterone, and the like, are used as a steroid, where glucosteroids are preferably used.

Food additives for use in the present invention can be prepared by a method similar to that used for the oral preparations of the present invention. Food additives are usually processed and manufactured into a form of, for example, powder, granules, pellets, tablets and various liquid agents by mixing or dissolving other food additives therein if necessary.

Foods and drinks for use in the present invention can be processed and manufactured by a commonly adopted method for producing foods and drinks except that a plant body of a plant that belongs to the genus Matricaria or Anthemis, the extract of the plant body or a food additive as herein described is added therein.

Foods and drinks for use in the present invention can also be produced by the methods including the following examples: granulation methods such as fluidized-bed granulation, agitating granulation, extrusion granulation, tumbling granulation, airflow granulation, compression-molding granulation, crushing granulation, spray granulation and injection granulation; coating methods such as pan coating, fluidized-bed coating and dry coating; expansion methods such as puff-dry, hypermoisture, foam-mat and microwave heating; extrusion methods using an extrusion granulator, an extruder, and the like.

Foods and drinks for use in the present invention may be served in any form including juice, carbonated drinks, teas, milk products such as lactic and bacteria drinks, fermented milk, frozen desserts, butter, cheese, yogurt, processed milk and nonfat milk, meat products such as ham, sausage and hamburger, fish cakes such as steamed fish pastes, fish sausages and fried fish pastes, egg products such as baked egg-rolls with soup stock flavor and steamed egg puddings, confectioneries such as cookies, jelly, chewing gums, candies and snack foods, breads, noodles, pickles, smoke foods, dry foods, foods boiled down in soy sauce, salt-cured foods, soups, seasonings and the like.

Further, foods and drinks for use in the present invention may be served in any form including, for example, powder foods, sheet-like foods, bottled foods, canned foods, retort foods, capsule foods, tablet-like foods, fluid foods, tonic drinks or the like.

Foods and drinks for use in the present invention can be used as foods and drinks such as health foods or functional foods or the like, having itch-relieving or antipruritic activity.

Foods and drinks or food additives for use in the present invention may be added with food additives that are commonly used for foods and drinks, such as, for example, a sweetener, colorant, preservative, thickening stabilizer, anti-oxidant, color brightener, bleach, fungicide, gum base, bittering agent, enzyme, brightener, acidifier, seasoning, emulsifier, strengthener, an agent for manufacturing, flavor, spice extract and the like.

A plant body of a plant that belongs to the genus Matricaria or Anthemis, the extract of the plant body or a food additive is added to foods and drinks for use in the present invention with an amount appropriately selected depending on the kinds of food and drink, on the effect expected by the intake of the foods and drinks, and so on. However, a plant body of a plant that belongs to the genus Matricaria or Anthemis is added to the foods or drinks with the resulting content of usually 0.1 to 100%, preferably 1 to 100% and more preferably 10 to 100% in dry weight of a plant body of a plant that belongs to the genus Matricaria or Anthemis. In the case where the extract of a plant body is added, an extract obtained from the plant body in dry weight within the above-mentioned range may be added.

Although the intake amount of foods and drinks differs depending on the form of intake, age and weight of an intaker, and so on, an intake amount per day for an adult is usually 0.1 to 5000 g, preferably 1 to 500 g in dry weight a plant body of a plant that belongs to the genus Matricaria or Anthemis, which is taken either once or multiple times a day. In the case where the extract of a plant body is used, an extract obtained from a plant body with dry weight within the above-mentioned range may be taken. There is no particular limitation to a period of intake, however, the period is usually from 1 day to 1 year, preferably 1 week to 3 months.

Feed additives for use in the present invention can be prepared in a similar manner as for preparing the oral preparations hereinbefore described. Feed additives are usually processed and manufactured into a form of, for example, powder, granules, pellets, tablets and various liquid agents by mixing or dissolving other feed additives therein if necessary.

Feeds for use in the present invention may be any feed including feed for pets such as dogs, cats, rabbits and mice; feed for livestock such as cows and pigs; feed for fowls such as chicken and turkey; feed for cultured fish such as sea-breams and yellowtails; feed for reptiles such as lizards, crocodiles and iguanas; feed for amphibias such as frogs, and the like, as long as the feed has itch-relieving or antipruritic activity against mammals, avians, reptiles, amphibias, pisces and the like.

Feeds for use in the present invention can be processed and manufactured by a common method for producing feed except that a plant body of a plant that belongs to the genus Matricaria or Anthemis, the extract of the plant body or a feed additive of the present invention is added to the feed.

Examples of feeds include grains, brans, vegetable oil cakes, animal feeds, other feeds, purified products, or the combination of these, and the like.

Grains include, for example, milo, wheat, barley, oats, rye, brown rice, buckwheat, foxtail millet, millet, green millet, corn, soy and the like.

Brans include, for example, rice bran, defatted rice bran, bran, powder, wheat, embryo, wheat bran, pellet, corn bran, corn embryo and the like.

Vegetable oil cakes include, for example, soy oil cake, soy flour, flax seed oil cake, cotton seed oil cake, peanut oil cake, suflower oil cake, coconut oil cake, palm oil cake, sesame oil cake, sunflower oil cake, rapeseed oil cake, kapok oil cake, mustard oil cake and the like.

Animal feed include, for example, fish meal such as white fish meal, imported meal, whole meal, brown fish meal, and fish soluble, meat meal, meat and bone meal, blood meal, degraded hair, bone meal, livestock-treatment byproducts, feather meal, silkworm feed, dry milk, casein, dried whey and the like.

Other feeds include the leaf and stem of plants such as alfalfa, haycube, alfalfa-leaf meal and pseudo-acacia powder; corn-processing byproducts such as corn gluten, meal, corn gluten feed and corn steep liquor; starch products such as starch; fermented products such as yeast, brewer's grain, malt root, alcohol cake and soy-sauce cake; agricultural byproducts such as citrus-product cake, bean curd cake, coffee cake and cocoa meal; cassava; horsebean; guar meal; seaweed; euphausiid; spirulina; chlorella; mineral substances, and the like.

Purified products include proteins such as casein and albumin, amino acids, starch, saccharides such as cellulose, sucrose and glucose, minerals, vitamins and the like.

Feeds for use in the present invention can be produced by, for example, granulation methods such as fluidized-bed granulation, agitating granulation, extrusion granulation, tumbling granulation, airflow granulation, compression-molding granulation, crushing granulation, spray granulation and injection granulation; coating methods such as pan coating, fluidized-bed coating and dry coating; expansion methods such as puff-dry, hypermoisture, foam-mat and microwave heating; extrusion methods using an extrusion granulator, an extruder, and the like.

A plant body of a plant that belongs to the genus Matricaria or Anthemis, the extract of the plant body or a feed additive is added to feeds hereinbefore described with an amount appropriately selected depending on the kinds of feeds, on the effect expected by the intake of the feeds, and so on. However, a plant body of a plant that belongs to the genus Matricaria or Anthemis is added to the feeds with the resulting content of usually 0.1 to 100%, preferably 1 to 100% and more preferably 10 to 100% in dry weight of a plant body of a plant that belongs to the genus Matricaria or the genus Anthemis.

Although the intake amount of feeds for use in the present invention differs depending on the form of intake, the species of an animal taking the feed, age and weight of an animal taking the feed, or the like, an intake amount per day for an animal is usually 0.1 mg to 150 g/kg, preferably 1 mg to 15 g/kg in dry weight of a plant body of a plant that belongs to the genus Matricaria or Anthemis, which is taken either once or multiple times a day. In the case where the extract of a plant body is used, an extract obtained from a plant body with dry weight of within the above-mentioned range may be taken. There is no particular limitation to the period of intake, however, the period is usually from 1 day to 1 year, preferably 1 week to 3 months.

The test examples are shown below.

Test Example 1 Suppressing effect on scratching behavior (1)

100% (v/v) methanol fraction of the chamomile extract prepared in Example 5 (hereinafter simply referred to as the fraction of chamomile extract) was dissolved in a solvent [dimethylsulfoxide (hereinafter abbreviated to DMSO):

polyoxyethylene sorbitan monooleate: saline = 1:1:8] to 25 mg/ml. Then by using this solution, the fraction of chamomile extract was intraperitoneally administered to 16 ddY mice (4 to 5-week-old, weight of about 20 g, male, purchased from Japan SLC, Inc.) at 100 mg/kg. Then Compound 48/80(Sigma) was dissolved in saline to 0.125 mg/ml, which was subcutaneously administered to the dorsorostral parts of 8 out of the 16 mice at 0.5 mg/kg (20$\mu$g/mouse) 20 minutes after the administration of the fraction of camomile extract. Scratching behavior of the mice for 30 minutes after the administration was observed. In addition, the remaining 8 mice were used to observe the effect on spontaneous motor activity using a spontaneous motor activity measurement system SUPERMEX (Muromachi Kikai Co. Ltd.) which employs a passive infrared sensor.

The scratching behavior and the spontaneous motor activity of the two 8-mice groups were observed in a similar manner as in the above except that the solvent (DMSO: polyoxyethylene sorbitan monooleate: saline - 1:1:8) alone was used instead of the solution in which the fraction of chamomile extract is dissolved.

The mice were pre-bred for 7 days or longer under free feeding with the standard feed (powder of the solid feed CE-2 for breeding and reproduction of mice, rats and hamsters: CLEA Japan, Inc.) and conditioned to the environment before being tested. They were bred in plastic cages (21 cm width $\times$ 31 cm depth $\times$ 14 cm height: Natsume Seisakusho Co., Ltd.) at 1 mouse/cage with a recycled paper floor mat PC (CLEA Japan, Inc.), in the breeding room where the condition was kept at temperature of 22 $\pm$ 2°C, moisture of 35 $\pm$ 15%, bright cycle of 12 hours/dark cycle of 12 hours.

The results are shown in Table 1. The determination results are shown by Mean $\pm$ standard error (N = 8) and t-test was conducted for the comparative test between the two groups. The difference was judged as significant with the risk rate of less than 5%.

Table 1

| Treatment | Counts of scratching behavior (count/ 30 min.) | Spontaneous motor activity (count/30 min.) |
| --- | --- | --- |
| Solvent alone | 83.4 $\pm$ 5.4 | 5970.5 $\pm$ 335.1 |
| Fraction of the chamomile extract | 23.4 $\pm$ 10.2 | 4522.5 $\pm$ 812.3 |

As shown in Table 1, no significant difference was observed between the 2 groups concerning the spontaneous motor activity. However, the counts of scratching behavior of the group intraperitoneally administered with the fraction of camomile extract were significantly suppressed by 71.2% (p < 0.01) compared to the counts of the group administered with the solvent (DMSO: polyoxyethylene sorbitan monooleate: saline = 1:1:8) alone. This reveals the effect of the fraction of camomile extract to suppress itch because the scratching behavior counts have been suppressed by the administration of the fraction of camomile extract without affecting the spontaneous motor activity.

Test Example 2 Suppressing effect on scratching behavior (2)

12 ddY mice (4 to 5-week-old, male, Japan SLC, Inc.) were pre-bred for 3 days under free feeding with the standard feed (powder of the solid feed CE-2 for breeding and reproduction of mice, rats and hamsters: CLEA Japan, Inc.) followed by a one-day fasting. Then the following feeding test was carried out.

6 mice were bred for one week under free feeding with the standard feed containing the fraction of chamomile extract prepared in Example 5 by 1% (w/w). Subsequently, the mice were administered with Compound 48/80 at 0.5 mg/kg in a similar manner as in Test example 1, and the scratching behavior for 30 minutes after the administration was observed.

Scratching behavior of the other 6 mice were observed in a similar manner as in the above, except that the mice were free-fed with the standard feed alone instead of the standard feed containing the fraction of chamomile extract by 1% (w/w) (hereinafter referred to as the feed containing the fraction of chamomile extract).

The mice were bred in plastic cages (12.5 cm width $\times$ 20 cm depth $\times$ 11 cm height: Natsume Seisakusho Co., Ltd.) at 1 mouse/cage with a recycled paper floor mat PC (CLEA Japan, Inc.), in the breeding room where the condition was kept at temperature of 22 $\pm$ 2°C, moisture of 35 $\pm$ 15%, bright cycle of 12 hours/dark cycle of 12 hours. The mice were free-fed with the feed and tap water during the breeding period. The floor mats were replaced once every week.

The results are shown in Table 2. The determination results are shown by Mean $\pm$ standard error (N = 6) and t-test was conducted for the comparative test between the two groups. The difference was judged as significant with the risk rate of less than 5%.

Table 2

| Treatment | Counts of scratching behavior (count/30 min.) | Weight Increase (g/7 days) |
|---|---|---|
| Standard feed | 75.7 ± 15.4 | 6.8 ± 0.2 |
| Feed containing the fraction of chamomile extract | 27.8 ± 7.2 | 6.8 ± 0.3 |

As shown in Table 2, the group with the feed containing the fraction of chamomile extract did not show any significant difference in the weight increase during the breeding period compared to the group fed with the standard feed, however, their scratching behavior was suppressed by 63.3% (significant at P < 0.02).

Test Example 3 Suppressing effect on scratching behavior (3)

33 ddY mice (4 to 5-week-old, male, Japan SLC, Inc.) were pre-bred for 3 days under free feeding with the standard feed (powder of the solid feed CE-2 for breeding and reproduction of mice, rats and hamsters: CLEA Japan, Inc.) followed by a one-day fasting. Then the following feeding test was carried out. The mice were bred under the same condition as in Test Example 2.

6 mice were bred for 11 days under free feeding with the standard feed containing the water extract of chamomile prepared in Example 1 by 7.7% (w/w) (equivalent to 30% (w/w) on a powder basis of chamomile) (hereinafter referred to as MCHW-containing feed). Then, mice in each group were administered with Compound 48/80 at 0.75 mg/kg in a similar manner as in Test Example 1, and the scratching behavior of the mice for 30 minutes after the administration was observed.

The scratching behavior of 6 mice each (except the group fed with the standard feed consisting of 15 mice) were observed in a similar manner as in the above except that the groups were respectively free-fed with the standard feed containing the ethyl acetate extract of chamomile prepared in Example 1 by 1.2% (w/w) (equivalent to 30% (w/w) on a powder basis of chamomile) (hereinafter referred to as MCEA-containing feed), the feed containing the commercially available camomile purified oil (Chamomile oil, Ogawa & Co., Ltd.) by 1% (w/w) (equivalent to 30% (w/w) or more on a powder basis of chamomile) (hereinafter referred to as MCEO-containing feed), and the standard feed alone, instead of the MCHW-containing feed.

The determination results are shown by Mean ± standard error (N = 6-15) and Dunnett method was carried out for the comparative test between each feed group and the standard feed group. The difference was judged as significant with the risk rate of less than 5%.

The results of weight increase during the test period are shown in Table 3.

Table 3

| Treatment | Weight increase (g/11 days) |
|---|---|
| Standard feed | 13.3 ± 0.3 |
| MCHW-containing feed | 12.4 ± 0.9 |
| MCEA-containing feed | 13.7 ± 0.6 |
| MCEO-containing feed | 13.3 ± 0.9 |

As shown in Table 3, there was no significant difference in weight increase during the test period among the MCHW-containing feed group, the MCEA-containing feed group, the MCEO-containing feed group and the standard feed group.

Table 4 shows the results of the count of scratching behavior.

Table 4

| Treatment | Count of Scratching behavior (count/30 min.) |
|---|---|
| Standard feed | 105.6 ± 8.5 |
| MCHW-containing feed | 77.4 ± 16.1 |
| MCEA-containing feed | 57.6 ± 10.0 |
| MCEO-containing feed | 100.6 ± 11.6 |

As shown in Table 4, only MCEA-containing feed group showed suppression of the scratching behavior compared to the standard feed group by 45.5% (significant at P < 0.01). The MCHW-containing feed group did not show any significant difference compared to the standard feed group but its scratching behavior was suppressed by 26.7%.

Test Example 4 Suppressing effect on scratching behavior (4)

56 ddY mice (4.5-week-old, male, Japan SLC, Inc.) were pre-bred for 3 days under free feeding with the standard feed (powder of the solid feed CE-2 for breeding and reproduction of mice, rats and hamsters: CLEA Japan, Inc.) followed by a one-day fasting. Then the following feeding test was carried out. The mice were bred under the same condition as in Test Example 2.

16 mice were bred for 10 days under free feeding with the standard feed containing the ethanol extract of chamomile prepared in Example 15 by 3.9% (w/w) (equivalent to 30% (w/w) on a powder basis of chamomile) (hereinafter referred to as MCEt-containing feed). Then Compound 48/80 was administered to the mice at 0.50 mg/kg in a similar manner as in Test Example 1, and the scratching behavior for 30 minutes after the administration was observed.

The scratching behavior of mice was observed in a similar manner as in the above except that 16 mice were free-fed with the standard feed containing the distribution of ethyl acetate in the ethanol extract of chamomile prepared in Example 16 by 1.9% (w/w) (equivalent to 30% (w/w) on a powder basis of chamomile) (hereinafter referred to as MCEE-containing feed) and 24 mice were free-fed with the standard feed alone. The scratching behavior of the mice was then observed, instead of the MCEt-containing feed.

The determination results are shown by Mean $\pm$ standard error (N = 16-24) and Dunnett method was carried out for the comparative test between each feed group and the standard feed group. The difference was judged as significant with the risk rate of less than 5%.

The counts of scratching behavior and the weight increase at the counting of the scratching behavior of the mice are shown in Table 5.

Table 5

| Treatment | Counts of scratching behavior (count/30 min.) | Weight increase (g/10 days) |
|---|---|---|
| Standard feed | 94.1 $\pm$ 15.2 | 10.3 $\pm$ 0.3 |
| MCEt-containing feed | 71.9 $\pm$ 12.8 | 10.5 $\pm$ 0.5 |
| MCEE-containing feed | 53.7 $\pm$ 10.3 | 9.5 $\pm$ 0.4 |

As shown in Table 5, the scratching behavior of the MCEE-containing feed group was suppressed by 42.9% compared to that of the standard feed group (significant at P < 0.01). On the contrary, there was no significant difference in the weight increase among the feed groups.

Test Example 5 Suppressing effect on scratching behavior (5)

57 ddY mice (4.5-week-old, male, Japan SLC, Inc.) were pre-bred for 3 days under free feeding with the standard feed (powder of the solid feed CE-2 for breeding and reproduction of mice, rats and hamsters: CLEA Japan, Inc.) followed by a one-day fasting. Then the following feeding test was carried out. The mice were bred under the same condition as in Test Example 2.

13 mice were bred for 10 days under free feeding with the standard feed containing the supercritical extract of chamomile prepared in Example 8 by 0.70% (w/w) (equivalent to 30% (w/w) on a powder basis of chamomile) (hereinafter referred to as MCSCl-containing feed). Then Compound 48/80 was administered to the mice at 0.50 mg/kg in a similar manner as in Test Example 1 and the scratching behavior for 30 minutes after the administration was observed.

The scratching behavior of mice was observed similarly as in the above except that the mice were free-fed as follows instead of being free-fed with MCSC1-containing feed. 13 mice were free-fed with the standard feed containing the supercritical extract of chamomile prepared in Example 9 by 0.94% (w/w) (equivalent to 30% (w/w) on a powder basis of chamomile) (hereinafter referred to as MCSC2-containing feed), 7 mice were free-fed with the standard feed containing the supercritical extract of chamomile prepared in Example 10 by 1.05% (w/w) (equivalent to 30% (w/w) on a powder basis of chamomile) (hereinafter referred to as MCSC3-containing feed), 7 mice were free-fed with the standard feed containing the supercritical extract of chamomile prepared in Example 11 by 1.20% (w/w) (equivalent to 30% (w/w) on a powder basis of chamomile) (hereinafter referred to as MCSC4-containing feed), and 17 mice were free-fed with the standard feed alone.

The determination results are shown by Mean $\pm$ standard error (N = 7-17) and Dunnett method was carried out for the comparative test between each feed group and the standard feed group. The difference was judged as significant with the risk rate of less than 5%.

The counts of scratching behavior and the weight increase at the counting of the scratching behavior of the mice are shown in Table 6.

Table 6

| Treatment | Counts of Scratching behavior (count/30 min.) | Weight increase (g/10 days) |
|---|---|---|
| Standard feed | 85.5 ± 19.5 | 10.7 ± 0.3 |
| MCSC1-containing feed | 69.9 ± 11.2 | 11.2 ± 0.4 |
| MCSC2-containing feed | 64.6 ± 13.5 | 10.4 ± 0.4 |
| MCSC3-containing feed | 39.7 ± 9.21 | 11.4 ± 0.5 |
| MCSC4-containing feed | 63.9 ± 12.4 | 10.6 ± 0.8 |

As shown in Table 6, the scratching behavior of the MCSC3-containing feed group was suppressed by 53.6% compared to that of the standard feed group (significant at $P < 0.05$). MCSC1-, MCSC2- and MCSC4-containing feed groups showed no significant difference with the standard feed group, however, their scratching behaviors were suppressed by about 30%. On the contrary, there was no significant difference in the weight increase among the feed groups during the breeding period.

Test Example 6 Suppressing effect on scratching behavior (6)

27 ddY mice (4 to 5-week-old, male, Japan SLC, Inc.) were bred for 3 days under free feeding with the standard feed (powder of the solid feed CE-2 for breeding and reproduction of mice, rats and hamsters: CLEA Japan, Inc.) followed by a one-day fasting. Then the following feeding test was carried out.

9 mice were bred for 11 days under free feeding with the standard feed containing the ethyl acetate extract of camomile prepared in Example 6 by 0.41% (w/w) (equivalent to 10% (w/w) on a powder basis of chamomile) (hereinafter referred to as 0.41% MCEA-containing feed). Then Compound 48/80 was administered to the mice at 0.50 mg/kg in a similar manner as in Test Example 1 and the scratching behavior for 30 minutes after the administration was observed.

The scratching behavior of mice was observed similarly as in the above except that the mice were free-fed with the standard feed containing MCEA by 1.24% (w/w) (equivalent to 30% (w/w) on a powder basis of chamomile) (hereinafter referred to as 1.24% MCEA-containing feed) and the standard feed alone, instead of the 0.41% MCEA-containing feed.

The determination results are shown by Mean ± standard error (N - 7-9) and Dunnett method was carried out for the comparative test between each feed group and the standard feed group. The difference was judged as significant with the risk rate of less than 5%.

The counts of scratching behavior and the weight increase during the breeding period are shown in Table 7.

Table 7

| Treatment | Counts of Scratching behavior (count/30 min.) | Weight increase (g/11 days) |
|---|---|---|
| Standard feed | 151.7 ± 14.8 | 10.9 ± 0.2 |
| 0.41%MCEA-containing feed | 141.6 ± 16.8 | 11.8 ± 0.4 |
| 1.24%MCEA-containing feed | 93.4 ± 16.5 | 9.9 ± 0.5 |

As shown in Table 7, the scratching behavior of the 1.24% MCEA-containing feed group was suppressed by 31.1% compared to that of the standard feed group (significant at $P < 0.05$). On the contrary, there was no significant difference in the weight increase among the feed groups at the completion of the test.

Test Example 7 Suppressing effect on scratching behavior (7)

43 ddY mice (4 to 5-week-old, male, Japan SLC, Inc.) were bred for 3 days under free feeding with the standard feed (powder of the solid feed CE-2 for breeding and reproduction of mice, rats and hamsters: CLEA Japan, Inc.) followed by a one-day fasting. Then, the following feeding test was carried out.

8 mice were bred for 10 days under free feeding with the standard feed containing the ethyl acetate distribution in the ethanol extract prepared in Example 17 by 2.01% (w/w) (equivalent to 30% (w/w) on a powder basis of chamomile) (hereinafter referred to as feed containing the distribution of ethyl acetate in the ethanol extract). Then Compound 48/80 was administered to the mice at 16.3 μg/mouse (equivalent to the dose of 0.50 mg/kg administered to the following standard feed group) in a similar manner as in Test Example 1 and the scratching behavior for 30 minutes after the administration was observed.

The scratching behavior of mice was observed similarly as in the above except that the mice were free-fed with the following feeds instead of the feed containing the distribution of ethyl acetate in the ethanol extract: the standard feed containing the ethanol extract of water extraction residue prepared in Example 18 by 5.64% (w/w) (equivalent to 30% (w/w) on a powder basis of chamomile) (hereinafter referred to as feed containing the ethanol extract of water extraction residue); the standard feed containing the ethanol extract of the hot water extraction residue (I) by 5.09% (w/w) (equivalent to 30% (w/w) on a powder basis of chamomile (hereinafter referred to as feed containing the ethanol extract of the hot water extraction residue (I)); the standard feed containing the ethanol extract of the hot water extraction residue (II) by 3.65% (w/w) (equivalent to 30% (w/w) on a powder basis of chamomile) (hereinafter referred to as feed containing the ethanol extract of the hot water extraction residue (II)); and the standard feed alone.

The determination results are shown by Mean ± standard error (N = 8, N = 11 only for the standard feed group) and Dunnett method was carried out for the comparative test between each feed group and the standard feed group. The difference was judged as significant with the risk rate of less than 5%.

The counts of scratching behavior and the weight increase during the breeding period of the mice are shown in Table 8.

Table 8

| Treatment | Counts of scratching behavior (count/30 min.) | Weight increase (g/10 days) |
|---|---|---|
| Standard feed | 92.6 ± 20.1 | 13.0 ± 0.4 |
| Feed containing the distribution of ethyl acetate in the ethanol extract | 50.0 ± 6.1 | 12.1 ± 0.8 |
| Feed containing the ethanol extract of water extraction residue | 59.8 ± 11.2 | 10.3 ± 0.7 |
| Feed containing the ethanol extract of the hot water extraction residue (I) | 56.6 ± 10.4 | 11.4 ± 0.4 |
| Feed containing the ethanol extract of the hot water extraction residue (II) | 65.0 ± 17.7 | 12.1 ± 0.5 |

As shown in Table 8, the scratching behavior of the distribution of ethyl acetate in the ethanol extract feed group was suppressed by 46.0% compared to that of the standard feed group (significant at $P < 0.05$). The groups of the ethanol extract of water extraction residue, the ethanol extract of the hot water extraction residue (I) and (II) showed no significant difference when compared with the standard feed group, however, their scratching behaviors were suppressed by 35.4%, 38.9% and 29.8%, respectively. On the contrary, the groups of the ethanol extract of the hot water extraction residue (I) and (II) showed no significant difference in the weight increase when compared with the standard feed group. Although weight increase in the ethanol extract of water extraction residue group was significantly decreased compared to that of the standard feed group, the difference was 2.7 g which is not very large, and the weights of the two groups during the test were 30.0 ± 1.8 g and 32.5 ± 1.7 g, respectively.

Test Example 8 Suppressing effect on scratching behavior (8)

20 ddY mice (4 to 5-week-old, male, Japan SLC, Inc.) were pre-bred for 3 days under free feeding with the standard feed (powder of the solid feed CE-2 for breeding and reproduction of mice, rats and hamsters: CLEA Japan, Inc.) followed by a one-day fasting. Then the following feeding test was carried out.

8 mice were bred for 10 days under free feeding with the standard feed containing the 50% ethanol extract of chamomile (CHAMOLILLAE FLOR EXTR.S.SICC, Product No.085742, Flachsmann) by 5% (w/w). Subsequently, the mice were administered with Compound 48/80 at 25.4 μg/mouse in a similar manner as in Test Example 1, and the scratching behavior for 30 minutes after the administration was observed.

Scratching behavior of 12 mice were observed in a similar manner as in the above, except that the mice were free-fed with the standard feed alone instead of the standard feed containing the 50% ethanol extract of chamomile by 5% (w/w) (hereinafter referred to as chamomile extract-containing feed).

The mice were bred in plastic cages (12.5 cm width × 20 cm depth × 11 cm height: Natsume Seisakusho Co., Ltd.) at 1 mouse/cage with a floor mat (Paperclean,Sankyo Labo Service Co., Ltd.) being placed, in the breeding room where the condition was kept at temperature of 22 ± 2° C, moisture of 35 ± 15%, bright cycle of 12 hours/dark cycle of 12 hours. The mice were allowed to take feed and tap water freely during the breeding period. The floor mats were replaced once every 3 days.

The results are shown in Table 9. The determination results are shown by Mean ± standard error (N = 8-12) and t-test was conducted for the comparative test between the two groups. The difference was judged as significant with the risk rate of less than 5%.

Table 9

| Treatment | Counts of scratching behavior (count/30 min.) | Weight increase (g/10 days) |
|---|---|---|
| Standard feed | 119.6 ± 21.1 | 11.0 ± 0.5 |
| Chamomile extract-containing feed | 87.3 ± 16.4 | 10.2 ± 0.6 |

As shown in Table 9, the chamomile extract-containing feed group showed 27.0% suppression in the scratching behavior when compared with the standard feed group, although no significant difference was observed. There was no significant difference between the chamomile extract-containing feed group and the standard feed group in weight increase during the breeding period.

Test Example 9 Suppressing effect on scratching behavior (9)

24 ddY mice (4 to 5-week-old, male, purchased from Japan SLC, Inc.) were pre-bred for 3 days or more under free feeding with the standard feed (powder of the solid feed CE-2 for breeding and reproduction of mice, rats and hamsters: CLEA Japan, Inc.). The following test was conducted after the mice were conditioned to the environment.

24 mice were orally administered with the fraction of chamomile extract at 300 mg/kg in a form of a solution in which the ethyl acetate extract of chamomile prepared in Example 1 was dissolved in a solvent [ethanol: polyoxyethylene sorbitan monooleate: saline = 1:1:8] to 15 mg/ml. Two hours after the administration of the ethyl acetate extract of chamomile, Compound 48/80 (Sigma) was subcutaneously administered to the dorsorostral part of the mice at 20 μg/site by using the solution consisting of saline and Compound 48/80 dissolved in the saline to 0.2 mg/ml. Then the scratching behavior of the mice was observed for 30 minutes after the administration.

The scratching behavior of 24 mice was observed in a similar manner as in the above except that the solvent alone was administered instead of the solution in which the ethyl acetate extract of chamomile is dissolved.

The mice were bred in plastic cages (21 cm width × 31 cm depth × 14 cm height: Natsume Seisakusho Co., Ltd.) at 6 to 7 mice/cage with a recycled paper floor mat PC (CLEA Japan, Inc.), in the breeding room where the condition was kept at temperature of 22 ± 2° C, moisture of 35 ± 15%, bright cycle of 12 hours/dark cycle of 12 hours.

The results are shown in Table 10. The determination results are shown by Mean ± standard error (N = 24) and t-test was conducted for the comparative test between the two groups. The difference was judged as significant with the risk rate of less than 5%.

Table 10

| Treatment | Scratching behavior (count/30 min.) |
|---|---|
| Solvent alone | 96.1 ± 10.0 |
| Ethyl acetate extract of chamomile | 58.0 ± 8.1 |

As shown in Table 10, scratching behavior of the group which was orally administered with the ethyl acetate extract of chamomile was suppressed by 39.6%, compared to the group administered with the solvent alone (significant at p < 0.01).

Test Example 10 Relieving effect on atopic dermatitis (1)

NC/Nga mice (models for spontaneous development of atopic dermatitis: Sankyo Labo Service Co., Ltd.) are known to be infested with Mycoptes spp. when bred and reproduced under the normal environment. They undergo spontaneous development of dermatitis in a long-term breeding and are thought to be an animal model with similar clinical condition to that of human in many aspects.

NC/Nga mice (6-week-old, male), therefore, were pre-bred for 3 days under free feeding with the standard feed (powder of the solid feed CE-2 for breeding and reproduction of mice, rats and hamsters: CLEA Japan, Inc.) followed by a one-day fasting. Then, the following feeding test was carried out.

8 NC/Nga mice were divided into two groups. One of the groups served as the standard feed group and the other served as the group fed with the standard feed containing 30% (w/w) chamomile powder (hereinafter also referred to as chamomile, powder-containing feed). The chamomile powder-containing feed was prepared as follows. Chamomile (Lot. 539220, Takasago Medical Co., Ltd.), which had been dried, was subjected to micropowderization using a grinder (Rotor Speed Mill, Pulverisette 14: FRITSCH) to obtain the chamomile powder. Then the powder was evenly mixed with the standard feed to obtain the powder content of 30% (w/w).

The mice were bred under the normal environment, namely, in plastic cages (21 cm width × 31 cm depth × 14 cm height: Natsume Seisakusho Co., Ltd.) at 2 mice/cage with a recycled paper floor mat PC (CLEA Japan, Inc.), in the breeding room where the condition was kept at temperature of $22 \pm 2°$ C, moisture of $35 \pm 15$%, bright cycle of 12 hours/dark cycle of 12 hours. The mice were allowed to take feed and tap water freely. The floor mats were replaced once every week.

3 to 6 weeks after the start of breeding under the normal environment, scratched wounds, bleeding, loss of hair and the like were observed in the mice, which have arisen from their scratching behavior. Mice were bred until they were finally 15.5-week-old (108-day-old).

After the breeding period was finished, scores of the skin conditions were determined as an index for the scratching behavior. As shown in Table 11 the skin conditions were graded into four stages, i.e. no symptoms, mild, moderate and severe, which were scored from 0 to 3 accordingly, for five items, i.e "reddening/bleeding", "edema", "abrade/ulcer", "scab formation/dryness" and "loss of hair" based on the assessment standard of the clinical symptoms of atopic dermatitis.

The results are shown in Table 11.

Table 11

| | I. Reddening /bleeding | II. Edema | III. Abrade /ulcer | IV. Scab formation /dryness | V. Loss of hair | Total score |
|---|---|---|---|---|---|---|
| Stage 0 (No symptoms) | No symptoms | No symptoms | No symptoms | No symptoms | No symptoms | |
| Stage 1 (Mild) | A small clot behind the ear or under the eye. Just scab is found. | A little swelling of the ear and the eye. | A little scratched wound behind the ear and under the eye. | A little skin dropoff behind the ear. | Hair is rather thin on the neck and under the eye. | |
| Stage 2 (Moderate) | Blood -stain behind the ear and all around under the eye. | Fairly swollen face | Scratched wound behind the ear and under the eye. | Dropoff of the skin all around behind the ear. | Skin can be seen at the neck and under the eye. | |
| Stage 3 (Severe) | Bleeding also from the body. | Swelling all over the body. | Scratched wound in the flank as well and ulcer is found. | Skin of all over the hair-loss site is dropped off. | Skin can be seen in the wide range of the back and some parts have almost no hair. | |
| Standard feed | 0.7 | 1.8 | 1.7 | 1.8 | 22 | 8.2 |
| Chamomile powder-containing feed | 0 | 0.5 | 1.5 | 2 | 2.5 | 6.5 |

As shown in Table 11, the total of the average scores of the standard feed group was 8.2 and of the chamomile powder-containing feed group was 6.5 when the full score was 15 (5 itemsx3 points each). Skin symptoms tended to be relieved by the intake of the chamomile powder.

The average intake amount of feed in the standard feed group was 5.7 g and the average weight at the end of the test was 26.6 g. Whereas the average intake of feed in the chamomile powder-containing feed group was 5.5 g and the average weight at the end of the test was 25.3 g, meaning that there was no big difference between the two groups in the intake amount of feed and the average weight.

Further, for the chamomile powder-containing feed group, blood collected at the end of the breeding test was left for 30 minutes at room temperature then the blood was centrifuged for 5 minutes at 10,000 g at 4°C to collect the serum, and glutamic-oxaloacetic transaminase (hereinafter abbreviated to GOT) and glutamic pyruvic transaminase (hereinafter abbreviated to GPT) which are indicators for hepatopathy were determined by using a transaminase determination kit (Transaminase C II-Test Wako (POP/TOOS method): Wako Pure Chemicals Industries, Ltd.). The enzyme activity was calculated as IU (International Unit)/L = Karmen unit $\times$ 0.482.

The result showed that GOT and GPT in the serum were about 30 IU/L and about 15 IU/L, respectively, both levels were within the normal range, meaning that no hepatopathy was observed in mice fed with chamomile powder.

Test Example 11 Relieving effect on atopic dermatitis (2)

Number of NC/Nga mice per group was yielded to be 8 for the standard feed group, and 8 for the chamomile powder-containing feed group, the test was conducted in a similar manner as in Test Example 10, except that they were bred until 99-day-old.

After the breeding period was finished, scores for skin symptoms were determined as an index for the scratching behavior.

The assessment standard for the clinical symptoms of atopic dermatitis was altered from that used in Test Example 10 to the standard suitable for NC/Nga strain mice. In other words, among the assessment standards used in Test Example 10, "reddening/bleeding" and "scab formation/dryness" were removed from the standard, because the former is difficult to be distinguished from abrade and ulcer and the latter is mild in mice compared to human. Instead, "auricular edema" and "ear laceration" were added to the standard, with which symptoms due to scratching were most considerably observed and by which quantitative assessment could be made. Then, as shown in Table 12, skin symptoms for the five items, i.e. "auricular edema (auricular thickness)", "ear laceration (scratched wound to the auricle)", "edema (other than the auricle)", "abrade/ulcer (other than the auricle)" and "loss of hair", were graded into 4 stages, i.e. no symptoms, mild, moderate and severe, which were scored from 0 to 3 accordingly.

The results are shown in Table 12. The determination results are shown by Mean $\pm$ standard error (N = 8) and t-test was conducted for the comparative test between the two groups. The difference was judged as significant with the risk rate of less than 5%.

Table 12

| | I. Auricular edema | II. Ear laceration | III. Edema (other than ear) | IV. Abrade/ ulcer (other then ear) | V. Loss of hair | Total score |
|---|---|---|---|---|---|---|
| Stage 0 (No symptoms) | No symptoms | No symptoms | No symptoms | No symptoms | No symptoms | |
| Stage 1 (Mild) | +0.05 mm or less. | A little laceration of the auricle. | A little swelling of the eye. | A little scratched wound under the eye and on the back | Rather thin hair on the neck and under the eye. | |
| Stage 2 (Moderate) | +0.2 mm or less. | Part of the auricle is lost. | Fairly swollen face. | Scratched wound under the eye, in the flank and on the back, with bleeding. | Skin can be seen on the neck and under the eye. | |

(continued)

|  | I. Auricular edema | II. Ear laceration | III. Edema (other than ear) | IV. Abrade/ ulcer (other then ear) | V. Loss of hair | Total score |
|---|---|---|---|---|---|---|
| Stage 3 (Severe) | +02 mm or more. | The auricle is almost lost. | Swelling has completely hidden the eye. | Scratched wounds all over the body and ulcer is emerging. | Skin can be seen in the wide range and some parts have alomost no hair. |  |
| Standard feed | 2.4 ± 0.2 | 1.4 ± 0.5 | 1.1 ± 0.4 | 1.8 ± 0.4 | 1.5 ± 0.4 | 8.1 ± 1.7 |
| Chamomile powder- containing feed | 1.4 ± 0.3 | 0.9 ± 0.4 | 0.3 ± 0.2 | 1.0 ± 0.3 | 1.5 ± 0.2 | 5.0 ± 0.8 |

As shown in Table 12, the total of the average scores of the standard feed group was 8.1 and of the chamomile powder-containing feed group was 5.0 when the full score was 15 (5 items $\times$ 3 points each). Scores of skin symptoms were confirmed to decrease in general by the intake of the chamomile. Particularly as to the auricular edema and edemas other than in the ear, significant relieving effects ($P<0.01$, $P < 0.05$, respectively), were observed.

When Mice Mycoptes spp. was observed under the microscope, both standard feed group and the chamomile powder-containing feed group were infested by more than several tens of ticks per mouse, and there was no big difference between the two groups.

It is known that skin symptoms of the atopic dermatitis patients and NC/Nga mice exacerbate in accordance with the increase of the serum IgE levels. The serum IgE levels of the NC/Nga mice, therefore, were determined before and at the end of the test to analyze the anti-allergic action of chamomile.

As the results, the serum IgE level of NC/Nga mice was 3.6 ± 1.4 mg/ml before the development of dermatitis (before the test) whereas the levels were 75.2 ± 17.4 mg/ml for the standard feed group and 93.7 ± 37.0 mg/ml for the chamomile powder-containing feed group at the end of the test, both of which were high IgE levels and there was no big difference between the two groups.

Test Example 12 Suppressing effect on contact dermatitis

NC/Nga TndCrj mice (SPF (specific pathogen-free): NC/Nga mice bred and reproduced at Charles River Japan, Inc.) do not spontaneously develop dermatitis at all when bred and reproduced under the normal environment, but are animal models for spontaneous development of atopic dermatitis that have atopic constitution and easily develop dermatitis.

Therefore, the model test for contact dermatitis induced by Picryl Chloride (hereinafter abbreviated to PCl) was conducted as follows using NC/Nga TndCrj mice, with reference to the methods of Kitagaki et al. (J. Invest. Dermatol. 105, 749-755, 1995) and of Ohmori et al. (Folia Pharmacol. Japan 80, 261-270, 1980).

8 NC/Nga TndCrj mice were divided into two groups. One of the groups served as the standard feed group and the other as the group fed with the standard feed containing 30% (w/w) chamomile powder (hereinafter referred to as the chamomile powder-containing feed group). Feed containing chamomile powder was prepared in a similar method as in Test Example 10.

After breeding the mice for 10 weeks in a similar manner as in Test Example 10, 20 $\mu$l of 1% (w/v) PCl dissolved in olive oil was applied to the ear lobules of the mice (the first application of PCl) to induce inflammatory response. Thickness of the ear lobules were measured just before the first application of PCl and 24 hours later (one day after the first application of PC1) using a Digimatic Caliper (Mitsutoyo Co. Ltd.).

Further, 20 $\mu$l of 1% (w/v) PCl dissolved in olive oil was again applied to the ear lobules of the mice after 1, 2, 5 and 7 days. 5 days after the repeated applications for 5 times as described above (12 days after the first application of PCl), thickness of the ear lobules were measured. The increase in the thickness of the ear lobules were calculated by the following formula and shown by Mean ± standard error.

Increase in the thickness of the ear lobules = Thickness of the ear lobules one day (24 hours) or 12 days after the first application of PCl - Thickness of the ear lobules just before the first application of PC1.

The results are shown in Table 13.

Table 13

| Treatment | One day after the first application of PCl | 12 days after the first application of PCl |
|---|---|---|
| Standard feed | 0.03 ± 0.01 | 0.25 ± 0.03 |
| Chamomile powder-containing feed | 0.01 ± 0.00 | 0.13 ± 0.02 |

As shown in Table 13, by the intake of chamomile powder-containing feed, there was observed a tendency that the inflammation of the ear lobules were suppressed 1 day after the first application of PCl, and a significant suppressing effect was observed 12 days after the repeated applications (t-test, significant at $P < 0.05$).

Test Example 13 Suppressing effect on the scratching behavior

It is known that when 2,4-Dinitrofluorobenzene (hereinafter abbreviated to DNFB) is applied to the mice ear lobules which have been sensitized with the anti-DNP monoclonal IgE in advance, scratching behavior will be induced because Substance P or Neurokinin A and the like are released from sensory nerves. On the contrary, application of DNFB induces a considerable scratching behavior in NC/Nga TndCrj mice even without the sensitization by anti-DNP monoclonal IgE in advance. Consequently, the model test for scratching behavior induced by DNFB using NC/Nga TndCrj mice was conducted as follows with reference to the method of Veronesi et al. (Toxicology and Applied Pharmacology, 135, 258-267 (1995)).

10 NC/Nga TndCrj mice were divided into two groups; 6 mice into the standard feed group and 4 mice into the group fed with the standard feed containing 30% (w/w) chamomile powder (hereinafter referred to as the chamomile powder-containing feed group). The chamomile powder-containing feed was prepared in a similar method as in Test Example 9

After having been bred for 10 weeks similarly as in Test Example 10, the mice were applied with 5 μl of 0.75% (w/v) DNFB, which was dissolved in a solution consisting of acetone and olive oil at 4:1, on both of their ear lobules. The scratching behavior counts after 0, 30, 60, 90 and 120 minutes of the application were counted for 10 minutes each. The measurement results are shown by Mean ± standard error.

The results are shown in Table 14.

Table 14

| Time course after the DNFB application | Standard feed | Chamomile powder-containing feed |
|---|---|---|
| 0-10 min. | 28.4 ± 14.5 | 0.3 ± 0.3 |
| 30-40 min. | 39.2 ± 11.0 | 24.5 ± 9.4 |
| 60-70 min. | 27.6 ± 11.5 | 15.8 ± 6.6 |
| 90-100 min. | 48.0 ± 32.5 | 2.0 ± 1.4 |
| 120-130 min. | 12.4 ± 6.8 | 0.8 ± 0.5 |

As shown in Table 14, the DNFB-induced scratching behavior was suppressed in every time range by the intake of the chamomile| powder-containing feed. A significant suppressing effect was observed especially in the ranges of 90 to 100 minutes and 120 to 130 minutes (t-test, significant at $P < 0.05$).

Test Example 14 Suppressing effect on the sensory nerve stimuli

Capsaicin is known to induce pain, itch or inflammation by specifically acting on Aδ-fiber and C-fiber of the sensory nerves (Pharmacological Reviews, 43, 143-201 (1991)). A substance having itch-relieving or antipruritic activity can, therefore, be screened as a material specifically suppressing the sensory nerve stimulation by capsaicin.

On the other hand, the guinea pig bronchial smooth muscle is known to contract by either of the followings. Neuropeptides (hereinafter abbreviated to NP) such as Substance P and the like are liberated from sensory nerves, which are distributed in the bronchi, by the sensory nerve stimulation, which NP then bind to NP receptors on the bronchial smooth muscle. Acetylcholine (hereinafter abbreviated to Ach) is released from parasympathetic nerves by the parasympathetic nerve stimulation, which Ach then binds to Ach receptors on the bronchial smooth muscle.

Consequently, a test to suppress the sensory nerve stimulation was conducted as follows using the bronchial sections of guinea pigs in accordance with the method of Kobayashi et al. (Planta Medica, 66, 526-530 (2000)).

Carotid arteries of female Hartley guinea pigs (purchased from Charles River Japan, Inc.), 8-week-old, 420-470 g weight, were incised and exsanguinated, then both right and left bronchis were promptly extracted and 4 ring sections of about 2 to 3 mm diameter and 1.5 to 2 mm length were prepared. The bronchial sections were immediately immersed in a specimen vessel of a magnus system (Micro Easy Magnus UC-2, 2 ml specimen vessel×4: IWASHIYA KISHIMOTO MEDICAL INSTRUMENTS) filled with Krebs-Henseleit solution consisting of 119 mmol/L sodium chloride, 4.7 mmol/L potassium chloride, 2.5 mmol/L calcium chloride, 1.2 mmol/L potassium dihydrogen-phosphate, 1.2 mmol/L magnesium sulfate, 25 mmol/L sodium hydrogencarbonate and 11.7 mmol/L glucose. To the Krebs-Henseleit solution was added 5 $\mu$mol/L indomethacin (Funakoshi) in order to suppress the generation of prostaglandins in the bronchi and the mixture was kept at 37° C while aerating with a mixture gas of 95% oxygen and 5% carbon dioxide. The bronchial sections were fixed on hooks in the specimen vessel, yielded with tensility of around 0.3 g, left for 20 to 30 minutes, and stabilized until the change in tensility was no longer observed. The tensility was measured by conversion to electric pulses using a pressure transducer (Isometric Tranceducer UM-203, IWASHIYA KISHIMOTO MEDICAL INSTRUMENTS). When the bronchial sections were stabilized, the tensility was adjusted to 0.15 g, electric pulse stimuli were yielded (10 Hz, 35 V, 0.1 $\mu$sec × 80 times), and then two-phase contraction, i.e. temporal Ach contraction and persistent NP contraction, was confirmed to occur. The sections were again washed in a nutrient solution and stabilized for use in the following test.

2 $\mu$mol/L carbachol, a synthetic inducer of Ach, was added as a parasympathetic nerve stimulator to the bronchial sections, and the maximum contraction of the sections was measured.

Next, the sections were washed after the Ach contraction was measured and the fraction of the German camomile extract prepared in Example 5 and dissolved in DMSO was added to the sections to 100 mg/ml. After incubating for 15 minutes at 37° C, 2 $\mu$mol/L capsaicin (a concentration showing the maximum contraction equivalent to 2 $\mu$mol/L carbachol) was added to the sections as a sensory nerve stimulator, and the NP bronchial contraction rate was immediately measured.

The bronchial contraction rate due to Ach was measured in a similar manner as the above except that 2 $\mu$mol/L carbachol was added instead of capsaicin.

The bronchial contraction rate was calculated by dividing the contraction caused by capsaicin or carbachol after pretreating with the fraction of the camomile extract, by the maximum contraction of the sections measured prior to the test. The measured results were shown by Mean $\pm$ standard error (N = 4). The comparative test between the capsaicin-induced contraction suppression and the carbachol-induced contraction suppression was performed with t-test and the difference was judged as significant with the risk rate of less than 5%.

The results are shown in Table 15.

Table 15

| Treatment | Capsaicin-induced contraction (%) | Carbachol-induced contraction (%) |
|---|---|---|
| Fraction of the chamomile extract | 12.93 $\pm$ 2.26 | 80.08 $\pm$ 17.56 |

As shown in Table 15, the fraction of the chamomile extract had specifically suppressed capsaicin-induced contraction of the bronchial sections, i.e. the sensory nerve stimulation, of guinea pigs (significant at P < 0.05).

Test Example 15 Suppressing effect against the sneeze response and the nasal rubbing behavior

Chronic rhinitis such as pollinosis-derived rhinitis often causes not only sneezing and stuffy nose but also itch deep in the throat or nasal cavity. Besides, sneezing, as well as itch, is caused by the mild stimulation of sensory nerves in the nasal cavity.

Consequently, the test to confirm the suppressing effect against the sneeze response and the nasal rubbing behavior in the guinea pig models for capsaicin-induced rhinitis was carried out as follows.

10 Hartley guinea pigs (5 to 6-week-old, male, purchased from Japan SLC, Inc.) were pre-bred for more than 5 days under the environment kept at the condition of temperature of 19 to 25°C, moisture of 30 to 70% and bright cycle of 12 hours/dark cycle of 12 hours, where the mice were allowed to take the commercially available solid feed Labo G Standard (G-2-A: Nosan Corporation) and tap water freely. The following test was performed after the guinea pigs were conditioned to the environment.

50% ethanol extract of chamomile (CHAMOLILLAE FLOR BXTR. S. SICC, Product No.085742, Flachsmann) was dissolved in a solvent (DMSO: polyoxyethylene sorbitan monooleate: saline = 1:1:8) to 25 mg/ml. By using this solution, 50% ethanol extract of chamomile was intraperitoneally administered to the Hartley guinea pigs at 100 mg/kg. 1 hour after the administration of 50% ethanol extract of chamomile, 50 $\mu$L of 1 mmol/L capsaicin solution (dissolved in saline containing 5% (v/v) ethanol and 5% (v/v) polyoxyethylene sorbitan monooleate) was administered to the right nasal cavities to induce the response. Counts of the sneezing and the nasal rubbing behavior emerged 30 minutes after the administration to the nasal cavities were counted by eye-observation and recorded (chamomile extract-administered group).

The sneezing response and the nasal rubbing behavior of 10 guinea pigs were counted by eye-observation and recorded in the same manner as the above except that the solvent alone was intraperitoneally administered instead of the solution in which 50% ethanol extract of chamomile was dissolved (Control group).

Further, 50 μL saline containing 5% (v/v) ethanol and 5% (v/v) polyoxyethylene sorbitan monooleate instead of 1 mmol/L capsaicin solution was administered to the right nasal cavities. Then counts of the sneezing response and the nasal rubbing behavior emerged 30 minutes after the administration was counted by eye-observation and recorded (mock-stimulated group).

The results are shown in Table 16. The measured results were shown by Mean ± standard error (N = 10). Statistical analysis soft SAS (Release 6.12, SAS Institute) was used for statistical analysis and non-parametric test method was adopted since the data were in discrete variables. The comparison between the two groups (mock-stimulated group and the control group, the control group and the camomile extract-administered group) was performed by Wilcoxon Rank Sum Test and the difference was judged as significant at the risk rate of below 5%.

The suppression rate in the chamomile extract-administered group was calculated according to the following formula.

$$\text{Suppression rate(\%)} = \frac{(\text{Level of control}) - (\text{Level of camomile extract-administered group})}{(\text{Level of control}) - (\text{Level of mock-stimulated group})} \times 100$$

Table 16

| Treatment | Sneezing response (count/30 min.) | Nasal rubbing response (count/30 min.) |
|---|---|---|
| Mock-stimulation | 0.7 ± 0.4 | 1.0 ± 0.9 |
| Solvent alone | 12.8 ± 1.5 | 31.3 ± 7.2 |
| Fraction of chamomile extract | 7.6 ± 1.6 | 17.3 ± 2.4 |

As shown in Table 16, the capsicin stimulation had significantly induced the sneezing response and the nasal rubbing response in the group in which normal guinea pigs were administered with the solvent alone, compared to the mock-stimulated group (p < 0.001). In contrast to the capsicin-induced response in the normal guinea pigs, the sneezing response was significantly suppressed in the group administered intraperitoneally with 50% ethanol extract of chamomile compared to the suppression in the group administered with the solvent alone (control group) (suppressed by 43%; p < 0.05). The nasal rubbing behavior was also suppressed by 46%, though not significant.

Test Example 16 Suppression effect on the scratching behavior in pet animals.

Powder agent containing the ethanol extract of chamomile prepared in Example 31 was given to 22 patient dogs that had developed various dermatitis accompanied by itch such as atopic dermatitis, miliary dermatitis, pyoderma, eczema, lick dermatitis, chronic dermatitis, acute dermatitis and the like, as listed in Table 17, everyday for 5 to 105 days at 0.5 to 3.88 g/dog or 0.5 g/10 kg weight.

Table 17

| No. | Variants | Sex | Age (years) | Weight (kg) |
|---|---|---|---|---|
| 1 | Shiba | Female | 10.0 | 10.1 |
| 2 | Shiba | Male | 7.0 | 33.3 |
| 3 | Demiwolf | Male | 10.0 | 24.2 |
| 4 | Demiwolf | Male | Unknown | 19.4 |

(continued)

| No. | Variants | Sex | Age (years) | Weight (kg) |
|-----|----------|-----|-------------|-------------|
| 5 | Demiwolf | Male | 13.0 | 10.6 |
| 6 | Shih Tzu | Female | 10.0 | 7.9 |
| 7 | Shih Tzu | Sterilized female | 9.0 | 6.9 |
| 8 | L. retriever | Female | 5.0 | 26.6 |
| 9 | Shiba | Male | 2.0 | 12.3 |
| 10 | Shih Tzu | Male | 2.0 | 9.5 |
| 11 | Demiwolf | Female | 14.0 | 8.9 |
| 12 | Demiwolf | Female | 11.0 | 13.1 |
| 13 | West Highland White Terriers | Female | 3.6 | 8.5 |
| 14 | Demiwolf | Castrated male | 8.0 | 10.5 |
| 15 | Shiba | Female | 13.0 | 13.6 |
| 16 | Shiba | Female | 9.0 | 9.9 |
| 17 | Demiwolf | Female | 15.0 | 20.3 |
| 18 | Shetland Sheepdog | Male | 4.0 | 9.8 |
| 19 | Shih Tzu | Male | 9.0 | 8.8 |
| 20 | Shih Tzu | Female | 11.0 | 6.4 |
| 21 | Golden retriever | Female | 9.0 | 34.1 |
| 22 | Dalmatian | Female | 1.0 | 18.3 |

Each patient dog was orally administered with powder agent containing the ethanol extract of chamomile for the period of dose and the dose shown in Table 17. The numbers in the Table correspond to the numbers for the patient dogs listed in Table 18.

15 patient dogs, Nos. 4 to 14 and 19 to 22 in the Table were orally administered with powder agent containing the ethanol extract of chamomile.

5 patient dogs, Nos. 1 to 3, 15 and 16 in the Table, were the dogs that had been administered with 1 tablet (5 mg of active ingredient) a day of predonisolone (PS; preparation for internal application for human), a steroid, with no clear therapeutic effect. These dogs were orally administered with powder agent containing the ethanol extract of chamomile along with predonisolone.

2 patient dogs diagnosed as pyoderma, Nos. 17 and 18 in the Table, were orally administered with powder agent containing the ethanol extract of chamomile with simultaneous administration of a new quinolone anti-bacterial agent (Victas) for internal application.

Clinical condition was judged in four stages, i.e. severe, moderate, mild and no symptoms by totaling the five checking items of reddening, swelling, leachate, loss of hair and itch.

For administration effects, 5 items, i.e. reddening, swelling, leachate, loss of hair and itch were yielded as checking items and veterinarians totaled the variations in each item during the therapy period and the effect on antipruritus was judged in 5 stages, i.e. considerably effective, effective, rather effective, not effective and deteriorated.

The results are shown in Table 18.

Table 18

| No. | Diagnosis | Clinical condition | Period of dose | Dose (g/dog) | Effect | Simultaneously used medicine |
|-----|-----------|--------------------|----------------|--------------|--------|------------------------------|
| 1 | Atopic dermatitis | Severe | 30 | 0.5 | Considerably effective | 1 tablet(5mg) /day of PS1 for the first 15 days |

(continued)

| No. | Diagnosis | Clinical condition | Period of dose | Dose (g/dog) | Effect | Simultaneously used medicine |
|-----|-----------|--------------------|----------------|--------------|--------|------------------------------|
| 2 | Miliary dermatitis | Severe | 10 | 1.8 | Effective | 1 tablet(5mg) /day of PS1 |
| 3 | Pyoderma | Severe | 10 | 1.5 | Not effective | 1 tablet(5mg) /day of PS1 |
| 4 | Atopic dermatitis | Severe | 7 | 3.88 | Not effective | Nil |
| 5 | Atopic dermatitis | Moderate to Mild | 42 | 1-0.5 | Effective | Nil |
| 6 | Atopic dermatitis | Moderate to Mild | 28 | 0.5 | Rather effective | Nil |
| 7 | Atopic dermatitis | Moderate to Mild | 42 | 0.5 | Not effective | Nil |
| 8 | Atopic dermatitis | Moderate to Mild | 49 | 1-2 | Effective | Nil |
| 9 | Atopic dermatitis | Moderate to Mild | 21 | 1 | Not effective | Nil |
| 10 | Eczema | Moderate to Mild | 21 | 1 | Effective | Nil |
| 11 | Lick dermatitis | Moderate to Mild | 105 | 0.5 | Effective | Nil |
| 12 | Atopic dermatitis | Moderate to Mild | 42 | 0.5 | Effective | Nil |
| 13 | Atopic dermatitis | Moderate to Mild | 84 | 0.5-1 | Rather effective | Nil |
| 14 | Atopic dermatitis | Moderate to Mild | 84 | 0.5 | Rather effective | Nil |
| 15 | Atopic dermatitis | Moderate | 80 | 0.5 | Effective | 1 tablet(5mg) /day of PS1 for the first 10 days |
| 16 | Atopic dermatitis | Moderate | 90 | 0.5 | Effective | 1 tablet(5mg) /day of PS1 |
| 17 | Pyoderma | Mild | 10 | 1 | Rather effective | Anti-bacterial agent (40 mg) /day |
| 18 | Pyoderma | Mild | 10 | 0.5 | Rather effective | Anti-bacterial agent (40 mg) /day |
| 19 | Chronic dermatitis | Mild | 14 | 0.5 | Not effective | Nil |
| 20 | Chronic dermatitis | Mild | 5 | 0.5 | Not effective | Nil |
| 21 | Atopic dermatitis | Mild | 7 | 2 | Effective | Nil |
| 22 | Acute dermatitis | Mild | 7 | 1 | Considerably effective | Nil |

With regard to the administration effects described in Table 18, the effect on antipruritus for the patient dogs (22 dogs) that were orally administered with the ethanol extract of chamomile is shown in Table 19, the effect on antipruritus for the patient dogs (15 dogs) that were orally administered with the ethanol extract of chamomile alone is shown in Table 20, and the effect on the antipruritus for the patient dogs (5 dogs) that were simulataneously and orally administered with the ethanol extract of chamomile and predonisolone is shown in Table 21, respectively.

Table 19

| Considerably effective | Effective | Rather effective | Not effective | Deterio rated | More than effective | More than rather effective |
|---|---|---|---|---|---|---|
| 2 dogs | 9 dogs | 5 dogs | 6 dogs | 0 dog | 50.0% | 72.7% |

Table 20

| Considerably effective | Effective | Rather effective | Not effective | Deterio -rated | More than effective | More than rather effective |
|---|---|---|---|---|---|---|
| 1 dog | 6 dogs | 3 dogs | 5 dogs | 0 dog | 46.7% | 66.7% |

Table 21

| Considerably effective | Effective | Rather effective | Not effective | Deterio -rated | More than effective | More than rather effective |
|---|---|---|---|---|---|---|
| 1 dog | 3 dogs | 0 dog | 1 dog | 0 dog | 80.0% | 80.0% |

As shown in Tables 19 to 21, antipruritic effect was observed either when the ethanol extract of chamomile was orally administered alone or orally administered simultaneously with predonisolone to the dogs suffering from dermatitis which is accompanied with itch alone.

In addition, as shown in Table 21, when the dogs suffering from severe dermatitis with no clear therapeutic effect upon the administration of predonisolone alone were orally administered simultaneously with powder agent containing the ethanol extract of chamomile, 1 considerably effective case and 1 effective case were obtained. 2 effective cases were also obtained in the moderate dermatitis. Therefore, the effectiveness of oral administration of the ethanol extract of chamomile simultaneously with predonisolone was confirmed.

The examples are now shown in the following.

**Best Modes for Carrying Out the Invention**

Example 1

Distilled water (7 L) was added to crude powder of dried German chamomile (350 g) (Lot. 539220: Takasago Medical Co., Ltd.) and the extraction was carried out for 3 hours at 70°C by using an ultrasonic generator (Ultrasonic Cleaner, Model SUP-20: Shibata). This operation was repeated twice and the extract liquid (14 L) was obtained by filtering with a filter cloth (Miracloth: Hoechst AG.). The residue was further washed with distilled water (3 L). The extract liquid obtained and the washing solution were together concentrated under reduced pressure using an evaporator (TOKYO RIKAKIKAI CO. LTD.) and freeze-dried. Water extract of chamomile (89.4 g) was thus obtained.

Ethyl acetate extract of chamomile (14 g) was obtained in a similar manner as the above except that ethyl acetate was used instead of distilled water.

Example 2

Upon preparing the water extract of Example 1, the residue obtained after the last operation was added with 100% (v/v) ethanol (6 L) and extracted for 3 hours while undergoing ultrasonic treatment. This operation was repeated twice and an extract liquid (about 14 L) was obtained. The extract liquid was concentrated and evaporated with an evaporator (TOKYO RIKAKIKAI CO. LTD.) and ethanol extract from the residue of the water extract of chamomile (31.8 g) was obtained.

Example 3

Acetone extract from the residue of the water extract of chamomile (40 g) was obtained in a similar manner as in Example 2 except that acetone was used instead of ethanol.

Example 4

Distilled water (20 L) was added to crude powder of dried German chamomile (1 kg) (Lot. 539220: Takasago Medical Co., Ltd.) and the extraction was carried out for 30 minutes at 70° C while stirring. After repeating this operation twice, extract liquid (40 L) was obtained by filtering with a miracloth. The residue was further washed with distilled water (10 L). The extract liquid obtained and the washing solution were together concentrated under reduced pressure and freeze-dried, and water extract of chamomile (220 g) was obtained.
Ethanol extract (100 g) and ethyl acetate extract (30 g) were respectively obtained in a similar manner as the above operation except that ethanol and ethyl acetate were used instead of distilled water.

Example 5

German chamomile (1 kg) (Flower head, Lot. 539220: Takasago Medical Co., Ltd.) was added with 70% (v/v) acetone (20 L), then the extraction was carried out for 6 days at room temperature while stirring with a stirrer (Drive member: Scroller SCR-120, Stirring wing: stirring wing for tornade T-80, Iuchi Seieido Co., Ltd.). The extract liquid was filtered with a miracloth to obtain the extract liquid. 100% (v/v) acetone (18 L) was added to the residue and stirred similarly for 4 days for extraction. These extract liquids obtained by the two procedures together underwent evaporation under reduced pressure to remove acetone, after which the liquids were passed through a column filled with a hydrophobic adsorption resin (HP-20 DIAION, 2 L : Mitsubishi Chemical Corporation) to adsorb the active ingredients. The extract adsorbed was washed in distilled water and 33% (v/v) methanol, 66% (v/v) methanol, 100% (v/v) methanol and 100% (v/v) acetone were respectively eluted by 2 bed volumes each, which was then evaporated under reduced pressure. 2 fractions of 100% (v/v) methanol for 11.9 g (1.2% (w/w)) and 1 fraction of 100% (v/v) acetone for 20.2 g (2.0% (w/w)) were obtained as active fractions. These fractions were dealt as 100% (v/v) methanol fraction and 100% (v/v) acetone fraction of the hydrated acetone extract of chamomile, respectively.

Example 6

German chamomile (450 g) (Flower head, Lot. 449222: Takasago Medical Co., Ltd.) was extracted with ethyl acetate by a similar method as in Example 1. The extract liquid was evaporated under reduced pressure and ethyl acetate extract of German chamomile (18.66 g) was obtained.

Example 7

German chamomile (500 g) (Flower head, Lot. 449222: Takasago Medical Co., Ltd.) was extracted with cyclohexane by a similar method as in Example 1. The extract liquid was evaporated under reduced pressure and cyclohexane extract of chamomilel(20.0 g) was obtained.

Example 8

German chamomile (Flower head, Shihira Shoten) was dried for 12 hours at 40° C, then 3 hours at 60° C in a dry oven (Constant Temperature Oven, DN63H: Yamato Scientific Co., Ltd.). The dry matter obtained (1 kg) was roughly crushed using a food processor (PRO CHEF FOOD PROCESSOR, PS-300S: CHUBUKOKI Co., Ltd.). Crude powder of chamomile thus obtained (576.9 g) was filled in an extraction vessel of a supercritical extractor (4 L volume) (Mitsubishi Kakoki Kaisha, Ltd.). The extraction was carried out under the conditions of the extraction vessel pressure of 30 Mpa, the extraction vessel temperature of 40° C, the separation vessel pressure of 4 Mpa and the extraction vessel at 40° C, by circulating carbon dioxide (19.1 kg) which was the integrated volume in 2 hours. As a result, supercritical extract of chamomile (24.8 g) was obtained.

Example 9

A supercritical extract of chamomile was prepared as follows by a similar method as in Example 8 except that 2.2% (w/w) ethanol was added as an entrainer.
German chamomile (1 kg) (Flower head, Shihira Shoten) was dried for 12 hours at 40° C and 3 hours at 60° C in a dry oven (Constant Temperature Oven, DN63H: Yamato Scientific Co. , Ltd.) was roughly crushed using a food processor (PRO CHEF FOOD PROCESSOR, PS-300S: CHUBUKOKI Co., Ltd.). Crude powder of chamomile thus obtained (527.3 g) was filled in an extraction vessel (4 L volume) of a supercritical extractor (Mitsubishi Kakoki Kaisha, Ltd.). The extraction was carried out under the conditions of the extraction vessel pressure of 30 Mpa, the extraction vessel temperature of 40°C, the separation vessel pressure of 4 Mpa and the separation vessel temperature of 40° C, where 410 g integrated volume of ethanol was added while circulating 18.5 kg of carbon dioxide which was the integrated volume in 2 hours. The extract obtained was evaporated under reduced pressure and supercritical extract of chamomile (13.38 g) was obtained.

Example 10

A supercritical extract of chamomile was prepared as follows by a similar method as in Example 8 except that 6.5 (w/w) ethanol was added as an entrainer.

German chamomile (0.6 kg) (Flower head, Shihira Shoten) dried for 8 hours at 60° C in a dry oven (Constant Temperature Oven, DN63H: Yamato Scientific Co., Ltd.) was roughly crushed using a food processor (PRO CHEF FOOD PROCESSOR, PS-300S: CHUBUKOKI Co., Ltd.). Crude powder of chamomile thus obtained (569.8 g) was filled in an extraction vessel (4 L volume) of a supercritical extractor (Mitsubishi Kakoki Kaisha, Ltd.). The extraction was carried out under the conditions of the extraction vessel pressure of 30 Mpa, the extraction vessel temperature of 40°C, the separation vessel pressure of 4 Mpa and the separation vessel temperature of 40° C, where 1042.3 g integrated volume of ethanol was added while circulating 16.0 kg of carbon dioxide which was the integrated volume in 2 hours. The extract obtained was evaporated under reduced pressure and supercritical extract of chamomile (16.54 g) was obtained.

Example 11

A supercritical extract of chamomile was prepared as follows by a similar method as in Example 8 except that 88.23% (w/w) ethanol aqueous solution was added by 7.6% (w/w) as an entrainer.

German chamomile (0.6 kg) (Flower head, Shihira Shoten) dried for 8 hours at 60° C in a dry oven was roughly crushed using a food processor. Crude powder of chamomile thus obtained (582.4 g) was filled in an extraction vessel (4 L volume) of a supercritical extractor (Mitsubishi Kakoki Kaisha, Ltd.). The extraction was carried out under the conditions of the extraction vessel pressure of 30 Mpa, the extraction vessel temperature of 40° C, the separation vessel pressure of 4 Mpa and the separation vessel temperature of 40°C, where 1228.7 g integrated volume of ethanol aqueous solution was added while circulating 14.3 kg of carbon dioxide which was the integrated volume in 2 hours. The extract obtained was evaporated under reduced pressure and supercritical extract of camomile (23.3 g) was obtained.

Example 12

Roman chamomile (500 g) (Flower head, Shihira Shoten) was roughly crushed using a food processor. Distilled water (10 L) was added therein, which was extracted for 3 hours by an ultrasonic treatment. This operation was repeated twice and the extract liquid of 20 L in volume was obtained. After having been concentrated by using an evaporator, the extract liquid obtained was freeze-dried and the chamomile water extract (150 g) was obtained.

Ethanol extract (115 g) and ethyl acetate extract (64 g) of chamomile were obtained by the above-mentioned method except that ethanol (10 L) and ethyl acetate (10 L) were respectively added instead of distilled water (10 L).

Example 13

Roman chamomile (1 kg) (Flower head, Shihira Shoten) was roughly crushed using a food processor. Boiled distilled water (20 L) was added therein, which was extracted for 30 minutes and then filtered. The extract liquid obtained was concentrated with an evaporator and freeze-dried, and the water extract of Roman chamomile (250 g) was obtained.

Example 14

The fraction of chamomile extract was prepared by the following method.

Roman chamomile (1 kg) (Flower head, Shihira Shoten) was roughly crushed using a food processor. 70% (v/v) acetone (10 L) was added thereto and cooled overnight, and an ultrasonic treatment was then conducted for 1 hour to obtain the extract liquid.

The extract liquid was filtered and collected using a miracloth and 70% (v/v) acetone (10 L) was added to the residue and the extract liquid was similarly obtained as the above. After the secondary 70% (v/v) acetone extract liquid was filtered and collected using a miracloth, 100% (v/v) acetone (10 L) was added to the residue and cooled overnight, an extract liquid was then obtained by the 1-hour ultrasonic treatment. This operation was repeated to obtain an extract liquid. The extract liquids obtained by the 4 extraction procedures were combined together and the acetone was evaporated under reduced pressure, after which the extract solutions were passed through a column filled with a hydrophobic adsorption resin (1.4 L) (HP-20 DIAION: Mitsubishi Chemical Corporation) to adsorb the active ingredients.

The column was washed in distilled water and 33% (v/v) methanol, 66% (v/v) methanol, 100% (v/v) methanol and 100% (v/v) acetone were respectively added by 2 bed volumes each, and the active ingredients adsorbed were sequentially eluted and fractionated. The fractions were each concentrated and evaporated, and 100% (v/v) methanol-eluted fraction and 100% (v/v) acetone-eluted fraction were obtained as active fractions which were dealt as the fractions of chamomile extract after having been concentrated and evaporated using an evaporator.

Example 15

Ethanol (36 L) was added to crude powder of dried German chamomile (1.8 kg) (Flower head, Shihira Shoten) and the extraction was carried out for 3 hours at 58°C using an ultrasonic generator (Ultrasonic Cleaner, Model SUP-20: Shibata). This operation was repeated 3 times and an extract liquid of 108 L volume was obtained by filtering with a filter cloth (Miracloth: Hoechst AG.). The residue was further washed in ethanol (12 L). The extract liquid obtained and the washing solution was combined together and concentrated and dried under reduced pressure to obtain the ethanol extract of chamomile (235 g).

Example 16

The ethanol extract obtained in Example 15 (54.8 g) was placed in a mortar to which a small amount of distilled water and ethyl acetate was added and the extract was dissolved by pounding and pasting the mixture. The mixture was then transferred to a separatory funnel to which distilled water (0.6 L) and ethyl acetate (0.6 L) were added, then the funnel was well shaken and left for the solvent distribution. The water layer obtained was washed twice in ethyl acetate and evaporated together with the ethyl acetate layer under reduced pressure using an evaporator, and the ethyl acetate distribution of the ethanol extract of chamomile (26.45 g) was obtained.

Example 17

Ethanol (16 L) was added to powder of dried German chamomile (0.4 kg) (Flower head, Shihira Shoten) and extraction was carried out for 14 hours at 40°C using a stirrer (Drive member: Scroller SCR-120, Stirring wing: stirring wing for tornade T-80, Iuchi Seieido Co., Ltd.). This operation was repeated 3 times in total and an extract liquid in 24 L volume was obtained by filtering with a filter cloth (Miracloth: Hoechst AG.). The residue was further washed in ethanol (2 L). The extract liquid obtained and the washing solution were together concentrated under reduced pressure and freeze-dried to obtain the ethanol extract of chamomile (61.73 g).

The ethanol extract thus obtained (60.19 g) was placed in a mortar to which a small amount of distilled water and ethyl acetate was added and the extract was dissolved by pounding and pasting the mixture. The mixture was then transferred to a separatory funnel with 2 L volume, to which distilled water (0.7 L) and ethyl acetate (0.7 L) were added, and the funnel was shaken for 60 minutes. Then after insoluble matters were filtered with a Buchner funnel (Qualitative filter paper No.2, Advantec Toyo Kaisha, Ltd.), the funnel was left for solvent distribution to obtain the ethyl acetate layer. The water layer was further washed twice in ethyl acetate (0.7 L). All the ethyl acetate layers obtained were combined, and concentrated and evaporated using an evaporator to obtain the distribution of ethyl acetate in the ethanol extract of chamomile (26.17 g).

Example 18

Distilled water (6 L) was added to powder of dried German chamomile| (0.2 kg) (Flower head, Shihira Shoten) and extraction was carried out for 30 minutes at 25°C using a stirrer (Drive member: Scroller SCR-120, Stirring wing: stirring wing for tornade T-80, Iuchi Seieido Co., Ltd.). The extraction residue obtained was added with ethanol (4 L) and extracted for 14 hours at 40°C using a stirrer (Drive member: Scroller SCR-120, Stirring wing: stirring wing for tornade T-80, Iuchi Seieido Co., Ltd.). This operation was further repeated twice and an extraction liquid of 12 L volume was obtained by filtering with a filter cloth (Miracloth: Hoechst AG). The residue was further washed in ethanol (2 L). The extract liquid obtained and the washing solution were together concentrated under reduced pressure and the ethanol extract of water extraction residue of chamomile (37.6 g) was obtained.

A similar operation as the above was conducted except that extraction with distilled water for 30 minutes at 40°C and for 3 hours at 40°C were conducted instead of extraction with distilled water for 30 minutes at 25°C. The ethanol extract of the hot water extraction residue of chamomile (I) (33.9 g), and the ethanol extract of the hot water extraction residue of chamomile (II) (24.3 g) were obtained, respectively.

Example 19

Drink is produced according to the following formula.

| Water extraction of chamomile prepared in Example 1 | 49 g |
|---|---|
| Pinedex#3 (Matsutani Chemical Industry Co.,Ltd.) | 49 g |
| Ferric pyrophosphate (Ferric source) | 0.1 g |
| Phoscal EFC (Calcium source: Nikko Fine Products) | 1 g |
| Vitamin mix (Merck & Co., Inc.) | 1 g |

The above composition (20 g) are dispersed in water (180 ml) to make a drink.

Example 20

Carbonated drink (10 bottles) is produced according to the following formula.

| Ethanol extract of chamomile prepared in Example 2 | 30 g |
|---|---|
| Vitamin C | 1 g |
| Vitamin B1 | 5 mg |
| Vitamin B2 | 10 mg |
| Vitamin B6 | 25 mg |
| Liquid sugar | 150 g |
| Citric acid | 3 g |
| Fragrant | 1 g |

Water is added to make the total amount to 1000 ml.

Example 21

Tea drink (1000 ml) is produced according to the following formula.

| Ethanol extract of chamomile prepared in Example 12 | 30 g |
|---|---|
| Tea leaf | 15 g |

Eluted in 1000 ml of water.

Example 22

Cookies (30 pieces) are produced according to the following formula by a conventional method.

| Ethanol extract of chamomile prepared in Example 15 | 30 g |
|---|---|
| Soft flour | 100 g |
| Starch | 74 g |
| Water | 14 g |
| Baking powder | 2 teaspoons |
| Salt | 1/2 teaspoon |
| Egg | 1 egg |
| Butter | 80 g |
| Milk | 2 tablespoons |
| Honey | small amount |

### Example 23

Sandwich loaf (4 loaves) is produced according to the following formula by a conventional method.

| | |
|---|---|
| Ethanol extract of chamomile prepared in Example 4 | 60 g |
| Hard flour | 1 kg |
| Sugar | 50 g |
| Salt | 20 g |
| Powdered skimmed milk | 20 g |
| Shortening | 60 g |
| Yeast (active) | 30 g |
| Yeast food | 1 g |
| Water | 650 g |

### Example 24

Chewing gums (30 pieces) are produced according to the following formula by a conventional method.

| | |
|---|---|
| Supercritical extract of chamomile prepared in Example 10 | 1 g |
| Gum base | 25 g |
| Sugar | 63 g |
| Starch syrup | 10 g |
| Fragrant | 1 g |

### Example 25

Candies (20 pieces) are produced according to the following formula by a conventional method.

| | |
|---|---|
| Supercritical extract of chamomile prepared in Example 11 | 1 g |
| Sugar | 80 g |
| Starch syrup | 20 g |
| Fragrant | 0.1 g |

### Example 26

Marmalade is produced according to the following formula by a conventional method.

| | |
|---|---|
| Water extract of chamomile prepared in Example 12 | 7 g |
| Chinese citron peel | 500 g |
| Sugar | 200 g |
| Chinese citron juice | 1 Chinese citron |

### Example 27

Tablets (300 mg per tablet) are produced according to the following formula by a conventional method.

| | |
|---|---|
| Water extract of chamomile prepared in Example 4 | 150 mg |
| Lactose | 90 mg |
| Cornstarch | 30 mg |
| Synthetic aluminum silicate | 12 mg |
| Carboxymethyl cellulose calcium | 15 mg |
| Magnesium stearate | 3 mg |

Example 28

Powder agents (1000 mg per package) are produced according to the following formula by a conventional method.

| | |
|---|---|
| Ethanol extract of chamomile! prepared in Example 4 | 500 mg |
| Lactose | 300 mg |
| Cornstarch | 200 mg |

Example 29

Hard capsules (360 mg per capsule) are produced according to the following formula by a conventional method.

| | |
|---|---|
| Supercritical extract of chamomile prepared in Example 9 | 250 mg |
| Lactose | 60 mg |
| Cornstarch | 30 mg |
| Hydroxypropyl cellulose | 20 mg |

Lactose (60 mg) and cornstarch (30 mg) are added to the supercritical extract of chamomile prepared in Example 9 (250 mg) and they are mixed. Aqueous solution containing 20 mg hydroxypropyl cellulose is added to the mixture, which is then kneaded. Then granules are prepared by a conventional method using an extrusion granulator. Hard capsules are produced by filling the granules in gelatin hard capsules.

Example 30

Soft capsules (170 mg per capsule) are produced according to the following formula.

| | |
|---|---|
| Supercritical extract of chamomile prepared in Example 10 | 50 mg. |
| Soybean oil | 120 mg |

The supercritical extract of chamomile produced in Example 10 (50 mg) is added to soybean oil (120 mg) and they are mixed. Then soft capsules are produced by filling the mixture in soft capsules by a conventional method using a rotary die automatic molding machine.

Example 31

Light anhydrous silicate (6 g) is added to 50% ethanol extract of chamomile (2 kg) (CHAMOLILLAB FLOR EXTR.S.SICC, Product No.085742, Flachsmann) as an anti-absorbent. The mixture is then fully mixed and individually packed in laminate packages each for 0.5 g, and powder agent is produced.

Example 32

Microcrystal cellulose (3.7 kg), magnesium stearate (0.5 kg), talcum (0.75 kg) and silica aerogel (0.05 kg) are added to 50% ethanol extract of chamomile (5 kg) (CHAMOLILLAB FLOR EXTR.S.SICC, Product No.085742, Flachsmann). The mixture is then fully mixed and individually packed in laminate packages each for 0.5 g, and powder agent is produced.

**Industrial Applicability**

The present invention makes it possible to provide oral preparations, foods, drinks, feeds, food additives or feed additives having an itch-relieving or antipruritic activity.

**Claims**

**1.** The use of a plant body of a plant that belongs to the genus *Matricaria* or *Anthemis* or of an extract of the plant body in the preparation of a medicament for oral administration for treating pruritis.

2. The use as claimed in claim 1, wherein the plant that belongs to the genus *Matricaria* or *Anthemis* is chamomile.

3. The use as claimed in claim 1 or claim 2, wherein the plant that belongs to the genus *Matricaria* or *Anthemis* is German chamomile *(Matricaria chamomilla* LINNAEUS) or Roman chamomile (*Anthemis nobilis* L.).

4. The use as claimed in any one of claims 1 to 3, wherein the oral medicament is for the treatment of itch derived from atopic dermatitis or pollinosis.

5. The use as claimed in any one of claims 1 to 4, wherein the oral medicament is a food, drink or feed.

6. The use as claimed in any one of claims 1 to 4, wherein the oral medicament is a food additive or a feed additive.

**Patentansprüche**

1. Verwendung eines Pflanzenkörpers einer Pflanze, die zu der Gattung *Matricaria* oder *Anthemis* gehört, oder eines Extrakts des Pflanzenkörpers bei der Herstellung eines Arzneimittels für die orale Verabreichung zur Behandlung von Pruritus.

2. Verwendung nach Anspruch 1, wobei die Pflanze, die zu der Gattung *Matricaria* oder *Anthemis* gehört, Kamille ist.

3. Verwendung nach Anspruch 1 oder Anspruch 2, wobei die Pflanze, die zu der Gattung *Matricaria* oder *Anthemis* gehört, Deutsche Kamille *(Matricaria chamomilla* LINNAEUS) oder Römische Kamille *(Anthemis nobilis* L.) ist.

4. Verwendung nach einem der Ansprüche 1 bis 3, wobei das orale Arzneimittel für die Behandlung von Hautjucken ist, das von atopischer Dermatitis oder Pollinose herrührt.

5. Verwendung nach einem der Ansprüche 1 bis 4, wobei das orale Arzneimittel ein Nahrungsmittel, Getränk oder Futtermittel ist.

6. Verwendung nach einem der Ansprüche 1 bis 4, wobei das orale Arzneimittel ein Nahrungsmittelzusatz oder ein Futtermittelzusatz ist.

**Revendications**

1. Utilisation d'une matière végétale d'une plante qui appartient au genre *Matricaria* ou *Anthemis* ou d'un extrait de la matière végétale dans la préparation d'un médicament destiné à une administration orale pour le traitement de prurit.

2. Utilisation selon la revendication 1, dans laquelle la plante qui appartient au genre *Matricaria* ou *Anthemis* est la camomille.

3. Utilisation selon la revendication 1 ou la revendication 2, dans laquelle la plante qui appartient au genre *Matricaria* ou *Anthemis* est la camomille allemande *(Matricaria chamomilla* LINNAEUS) ou la camomille romaine (*Anthemis nobilis* L.).

4. Utilisation selon l'une quelconque des revendications 1 à 3, dans laquelle le médicament pour la voie orale est destiné au traitement de démangeaisons dues à une dermatite atopique ou une pollinose.

5. Utilisation selon l'une quelconque des revendications 1 à 4, dans laquelle le médicament pour la voie orale est un aliment, une boisson ou un aliment pour animaux.

6. Utilisation selon l'une quelconque des revendications 1 à 4, dans laquelle le médicament pour la voie orale est un additif alimentaire ou un additif aux aliments pour animaux.

## REFERENCES CITED IN THE DESCRIPTION

**Patent documents cited in the description**

- JP 2124237 A
- JP 3145430 A
- JP 9100236 A
- JP 6006525 B
- JP 06078727 A

**Non-patent literature cited in the description**

- Medical Dictionary, section "Pruritus. NANZANDO Co., Ltd, 1998
- Medical Dictionary, section "Itch. NANZANDO Co., Ltd, 1998
- **UKO HIGAKI.** Appendix to Experimental Medicine, Medical Term Library. *Allergy,* 1996, 128-129
- **KIYOSHI NISHIOKA.** *Text for Atopic Dermatitis,* 1995, 54-57
- Makino's Illustrated Encyclopedia of Japanese and Chinese Medical Plants in Colour. Hokuryukan Co., Ltd, 568
- 95 Application of Medical Herb. CMC Publishing Co., Ltd, 1995, 90-91
- **TSUJI et al.** *J. Soc. Cosmet. Chem. Japan,* 1992, vol. 25, 1-15
- **ANDERSON et al.** *Phytotherapy Research,* 2000, vol. 14, 452-456
- **KITAGAKI et al.** *J. Invest. Dermatol.,* 1995, vol. 105, 749-755
- **OHMORI et al.** *Folia Pharmacol. Japan,* 1980, vol. 80, 261-270
- **VERONESI et al.** *Toxicology and Applied Pharmacology,* 1995, vol. 135, 258-267
- *Pharmacological Reviews,* 1991, vol. 43, 143-201
- **KOBAYASHI et al.** *Planta Medica,* 2000, vol. 66, 526-530